# EUROPEAN PATENT APPLICATION

(11) **EP 4 837 572 A1**
(43) Date of publication of application: **11.11.2026**
(21) Application number: 25174784.6
(22) Date of filing: 07.05.2025
(51) Int. Cl.: A61K 31/4184, A61K 31/4188, A61K 31/438, A61K 31/496, A61K 31/53, A61P 19/02, A61P 29/00, A61P 37/00, C12N 5/0783

(54) **SMALL MOLECULES INHIBITING THE CD3E-LCK INTERACTION FOR USE IN THERAPY**

(71) Applicant: Albert-Ludwigs-Universität Freiburg, 79098 Freiburg (DE)
(72) Inventor: MINGUET, Susana, 79100 Freiburg (DE); WÖSSNER, Nadine, 79100 Freiburg (DE)
(74) Representative: Maiwald GmbH

(57) **Abstract**

The present application is in the field of modulating T cell activity and discloses a small molecule binding to the SH3 domain of the lymphocyte-specific protein tyrosine kinase (LCK), wherein the small molecule reduces or prevents the interaction of LCK with CD3ε; as well as compounds of formulae (I), (II) and (III), preferably for use in therapy, for use in treating a T cell involving autoimmune or inflammatory disease, or for use in treating a side effect associated with a T cell based immunotherapy. The present application further discloses an *in vitro* method of manufacturing a T cell comprising a chimeric antigen receptor (CAR) or a TCR-based chimeric receptor, comprising the step of contacting the T cell with a small molecule binding to the SH3 domain of the lymphocyte-specific protein tyrosine kinase (LCK), wherein the small molecule reduces or prevents the interaction of LCK with CD3ε; as well as with compounds of formulae (I), (II) and (III). Further, the use of the afore-mentioned small molecule as well as the compounds of formulae (I), (II) and (III) to prevent or reduce early exhaustion of a T cell, optionally a T cell comprising a chimeric antigen receptor (CAR) or a TCR-based chimeric receptor is disclosed herein.

## Description

### TECHNICAL FIELD

The present application is in the field of modulating T cell activity. Thus, the present application is concerned with a small molecule binding to the SH3 domain of the lymphocyte-specific protein tyrosine kinase (LCK), wherein the small molecule reduces or prevents the interaction of LCK with CD3ε, (i) for use in therapy, (ii) for use in treating a T cell involving autoimmune or inflammatory disease, or (iii) for use in treating a side effect associated with a T cell-based immunotherapy. The present application is further concerned with an *in vitro* method of manufacturing a T cell comprising a chimeric antigen receptor (CAR) or a TCR-based chimeric receptor, comprising the step of contacting the T cell with a small molecule binding to the SH3 domain of the lymphocyte-specific protein tyrosine kinase (LCK), wherein the small molecule reduces or prevents the interaction of LCK with CD3ε. Also, the present application relates to a use of a small molecule binding to the SH3 domain of the lymphocyte-specific protein tyrosine kinase (LCK), wherein the small molecule reduces or prevents the interaction of LCK with CD3ε, to prevent or reduce early exhaustion of a T cell, optionally a T cell comprising a chimeric antigen receptor (CAR) or a TCR-based chimeric receptor. Furthermore, the present application is concerned with compounds according to formulae (I), (II) and (III), as well as pharmaceutical compositions thereof and their medical use for treating (i) a T cell involving autoimmune or inflammatory disease or (ii) for use in treating a side effect associated with a T cell based immunotherapy. The present application is also concerned with an *in vitro* method of manufacturing a T cell comprising a chimeric antigen receptor (CAR) or a TCR-based chimeric receptor, comprising the step of contacting the T cell with the compounds according to formulae (I), (II) and (III); as well as the use of the compounds according to formulae (I), (II) and (III) to prevent or reduce early exhaustion of a T cell, optionally a T cell comprising a chimeric antigen receptor (CAR) or a TCR-based chimeric receptor.

### BACKGROUND OF THE INVENTION

The αβ T cell receptor (TCR) is the main receptor regulating T cell activation. It consists of a ligand-binding αβ heterodimer and the signal transducing CD3 dimers CD3ε:CD3δ, CD3ε:CD3γ and ζ:ζ. Upon binding to its cognate ligand, the major histocompatibility complex bound to an antigenic peptide (pMHC), the lymphocyte-specific protein tyrosine kinase (LCK) phosphorylates the immunoreceptor tyrosine-based activation motifs (ITAMs) of the CD3 and ζ chains. This leads to the recruitment and phosphorylation of downstream molecules and the initiation of TCR signaling.

LCK is a 56 kDa protein and belongs to the Src family of non-receptor protein kinases (SFK). It consists of an N-terminal site with a unique domain (UD), which interacts with the T cell co-receptors CD4 and CD8, a SH3 and a SH2 domains, a highly conserved kinase domain, and a short C-terminal tail (Carrera et al., 1995; Sicheri & Kuriyant, 1997).

The molecular mechanism on how the un-phosphorylated TCR interacts with LCK, leading to its initial activation, was recently unraveled, and a new motif within the cytoplasmic tail of CD3ε, the receptor kinase (RK) motif, which mediates the initial recruitment of LCK to the TCR via LCK's SH3 domain, was identified (Hartl et al., 2020). The RK motif is a non-canonical SH3-binding motif, which interacts with the RT loop and n-SRC loop of the SH3(LCK) domain. The residues of the loops are not conserved in other SFKs. Mutation of the RK motif leads to the disruption of its protein-protein interaction with LCK and thereby to reduced T cell activation (Hartl et al., 2020). Only two related RK motifs have been reported, one in the T cell adaptor protein SKAP1 (RKxx(Y)xxY) (Kang et al., 2000) and one in *Candida albicans* (Gkourtsa et al., 2016), highlighting the uniqueness of the LCK-RK interaction. The interaction of LCK with the TCR is further supported by the ionic interaction of LCK's UD with the basic-rich stretch (BRS) motif of CD3ε (Li et al., 2017), and by the recruitment of co-receptor-associated LCK (Horkova et al., 2023). While genetic mutations affecting these interactions have been shown to reduce T cell activation, specific inhibitors targeting LCK recruitment to the TCR do not exist thus far.

The important role of CD3ε in enhancing T cell-based immunotherapies is known. Thus, introducing CD3ε into a chimeric antigen receptor (CAR) construct, or fusing the tumor-targeting single-chain variable fragment to the CD3ε chain, generating a TCR fusion construct (TRuC), greatly increases the anti-tumor efficacy of these therapies (Baeuerle et al., 2019; Hartl et al., 2020; Salter et al., 2021; Velasco Cárdenas et al., 2023; Wu et al., 2020). However, while CAR T cell therapy is a major breakthrough in the treatment of cancer, patients still suffer from severe side effects, like cytokine release syndrome (CRS), neurotoxicity, or pre-mature exhaustion due to excessive activation of the CAR T cells.

LCK has emerged as major target for drug discovery, as small molecule inhibitors could be used as antiinflammatory agent in autoimmune diseases, graft-versus-host disease (GvHD), and transplant rejection. However, existing inhibitors exclusively target the kinase domain of LCK, which is highly conserved among the SKF family, making them unselective (Elkamhawy et al., 2021; Martin & Machacek, 2010).

In the light of the above, there is the need for small molecules that specifically bind to the SH3(LCK) domain and thereby disrupt its interaction with the RK motif of CD3ε (while avoiding unspecific off-target effects on other kinases), with the effect of controlling T cell activation in immune-mediated diseases and reducing side effects of T cell-based immunotherapy.

### SUMMARY OF THE INVENTION

The invention as disclosed in the present application solves the above need.

In a **first aspect,** the present application provides to a small molecule binding to the SH3 domain of the lymphocyte-specific protein tyrosine kinase (LCK), wherein the small molecule reduces or prevents the interaction of LCK with CD3ε, for use in therapy.

In a **second aspect,** the present invention provides a small molecule binding to the SH3 domain of the lymphocyte-specific protein tyrosine kinase (LCK), wherein the small molecule reduces or prevents the interaction of LCK with CD3ε, for use in treating a T cell involving autoimmune or inflammatory disease.

In a preferred embodiment of the second aspect, the small molecule is for use in treating a T cell-driven autoimmune or inflammatory disease.

In another preferred embodiment of the second aspect, the autoimmune or inflammatory disease is selected from the group consisting of rheumatoid arthritis, psoriasis, multiple sclerosis, atherosclerosis, graft-versus-host disease, allograft rejection, asthma, type 1 diabetes mellitus, vitiligo, celiac disease, Graves' disease, Hashimoto's thyroiditis, Addison's disease, systemic lupus erythematosus, Sjögren's Syndrome, systemic sclerosis, inflammatory bowel disease including Crohn's Disease and ulcerative colitis, Lichen Planus, atopic dermatitis, autoimmune hemolytic anemia, immune thrombocytopenic purpura (ITP), alopecia areata, autoimmune uveitis, sarcoidosis and ankylosing spondylitis.

In an embodiment of the first and the second aspect, the CD3ε is comprised in a T cell receptor complex (TCR).

In another embodiment of the first and second aspect, the CD3ε does not carry a mutation affecting the interaction with LCK.

In yet another embodiment of the first and second aspect, the CD3ε is wild-type.

In a **third aspect,** the present application provides a small molecule binding to the SH3 domain of the lymphocyte-specific protein tyrosine kinase (LCK), wherein the small molecule reduces or prevents the interaction of LCK with CD3ε, for use in treating a side effect associated with a T cell-based immunotherapy.

In an embodiment of the third aspect, the side effect associated with a T cell-based immunotherapy is caused by an overactivation of the T cell used in the immunotherapy.

In a preferred embodiment of the third aspect, the side effect associated with a T cell-based immunotherapy is selected from cytokine release syndrome, neurotoxicity and graft-versus-host disease.

In an embodiment of the third aspect, the T cell-based immunotherapy is a chimeric antigen receptor (CAR) T cell immunotherapy.

In another embodiment of the third aspect, the T cell-based immunotherapy is a TCR-based chimeric receptor T cell immunotherapy. It can be preferred in this embodiment that the TCR-based chimeric receptor is selected from the group consisting of a TCR fusion construct (TRuC), a TCR containing an antibody-derived Fab fragment (AbTCR), a synthetic TCR and antigen receptor comprising V regions of an antibody (STAR), a HLA-independent T cell receptor comprising V regions of an antibody (HIT), and a

T cell antigen coupler comprising an antigen-directed ScFv fused to an anti-CD3 scFv (TAC).

In an embodiment of the third aspect, the CD3ε is comprised in the chimeric antigen receptor (CAR) or the TCR-based chimeric receptor.

In a **fourth aspect,** the present application provides an *in vitro* method of manufacturing a T cell comprising a chimeric antigen receptor (CAR) or a TCR-based chimeric receptor, comprising the step of contacting the T cell with a small molecule binding to the SH3 domain of the lymphocyte-specific protein tyrosine kinase (LCK), wherein the small molecule reduces or prevents the interaction of LCK with CD3ε. Typically, the T cell underlying the T cell comprising a CAR or a TCR-based chimeric receptor (i.e., the T cell that has not yet been genetically modified to express a CAR or a TCR-based chimeric receptor) is obtained from a patient or a healthy donor compatible with the patient to be treated.

In an embodiment of the fourth aspect, the method further comprises the steps of (i) genetically modifying a T cell to express a CAR or a TCR-based chimeric receptor and (ii) expanding the so modified T cell. Typically, genetically modifying the T cell to express a CAR or a TCR-based chimeric receptor is carried out by introducing a nucleic acid expressing the CAR or the TCR-based chimeric receptor into the T cell, wherein the introduction preferably results in a stable integration of the nucleic acid expressing the CAR into the genome of the T cell.

In a preferred embodiment of the fourth aspect, the T cell is contacted with the small molecule after having been genetically modified, preferably about one to about three days after having been genetically modified, most preferably about two days after having been genetically modified.

In another preferred embodiment of the fourth aspect, the T cell is contacted with the small molecule when expanding the genetically modified T cell (wherein this expansion is cytokine-mediated, e.g., via IL2 or a combination of IL7 and IL-15), preferably wherein the T cell is contacted with the small molecule when expanding the genetically modified T cell during a period of from about 3 days to about 2 weeks, more preferably during a period of from about 5 days to about 12 days, most preferably for a period of about 10 days. It is noted that the period, in which the T cell is expanded and contacted with the small molecule, can depend on the specific T cell obtained from a patient, and may thus vary from donor to donor, preferably within the afore-mentioned periods.

In a preferred embodiment of the fourth aspect, the contacting is carried out by adding the small molecule to the medium, in which the T cell is incubated.

In a **fifth aspect,** the present application provides a use of a small molecule binding to the SH3 domain of the lymphocyte-specific protein tyrosine kinase (LCK), wherein the small molecule reduces or prevents the interaction of LCK with CD3ε, to prevent or reduce early exhaustion of a T cell, optionally a T cell comprising a chimeric antigen receptor (CAR) or a TCR-based chimeric receptor. It can be preferred that the TCR-based chimeric receptor is selected from the group consisting of a TCR fusion construct (TRuC), a TCR containing an antibody-derived Fab fragment (AbTCR), a synthetic TCR and antigen receptor comprising V regions of an antibody (STAR), a HLA-independent T cell receptor comprising V regions of an antibody (HIT), and a T cell antigen coupler comprising an antigen-directed ScFv fused to an anti-CD3 scFv (TAC). In an embodiment, the CD3ε is comprised in the chimeric antigen receptor (CAR) or the TCR-based chimeric receptor.

The following embodiments relate to the first, the second, the third, the fourth, and the fifth aspect.

In an embodiment, the small molecule reduces or prevents the interaction of LCK with the cytoplasmic tail of CD3ε.

In another embodiment, the small molecule reduces or prevents the interaction of LCK with the receptor kinase (RK) motif of CD3ε.

In another embodiment, the CD3ε is unphosphorylated.

In yet another embodiment, the small molecule binds to the RT loop and/or the n-SRC loop of the SH3 domain of LCK.

In a preferred embodiment, the small molecule exhibits an IC50 of < about 20 µM in an *in vitro* T cell proliferation inhibition assay. It can be particularly preferred that the small molecule exhibits an IC50 of < about 10 µM in an *in vitro* T cell proliferation inhibition assay.

T cell proliferation inhibition can, e.g., be determined by flow cytometry. Briefly, in a T cell proliferation inhibition assay relying on flow cytometry, T cells are (i) stimulated (e.g., with an anti-CD3ε antibody, an anti-TCR antibody, a combination of an anti-CD3ε antibody and an anti-CD28 antibody, a combination of an anti-TCR antibody and an anti-CD28 antibody, 12-myristate 13-acetate (PMA), ionomycin, phytohaemagglutinin, a combination of PMA and ionomycin, or peptide-loaded antigen presenting cells (all of the afore-mentioned agents optionally in the presence of IL-2), or IL-2, (ii) stained with a dye (e.g., with CellTrace Violet), (iii) incubated for a period of from about 2 days to about 10 days (such as, e.g., for 2, 3, 4, 5, 6, 7, 8, 9 or 10 days), and (iv) assayed via flow cytometry as regards (a) cell number, (b) percent dye-positive cells relative to all cells, or (c) loss of dye intensity in the overall cell population. If necessary, a control group that is not contacted with the small molecule is included in the assay, which serves as comparative group. If necessary, a control group of unstimulated cells is included in the assay, which serves as comparative group.

Alternatively, T cell proliferation inhibition can, e.g., be determined by cell counting. Briefly, in a T cell proliferation inhibition assay relying on counting, T cells are (i) stimulated (e.g., with an anti-CD3ε antibody, an anti-TCR antibody, a combination of an anti-CD3ε antibody and an anti-CD28 antibody, a combination of an anti-TCR antibody and an anti-CD28 antibody, 12-myristate 13-acetate (PMA), ionomycin, phytohaemagglutinin, a combination of PMA and ionomycin, or peptide-loaded antigen presenting cells (all of the afore-mentioned agents optionally in the presence of IL-2) or IL2, (ii) incubated for a period of from about 2 days to about 10 days (such as, e.g., for 2, 3, 4, 5, 6, 7, 8, 9 or 10 days), and (iii) counted, wherein typically a control group that is not contacted with the small molecule is compared to a group that is contacted with the small molecule and the inhibition of proliferation is determined by this comparison.

Yet alternatively, T cell proliferation inhibition can, e.g., be determined by a radioactive thymidine incorporation assay. Briefly, in a T cell proliferation inhibition assay relying on thymidine incorporation, T cells are (i) incubated in the presence of radioactive thymidine (3H-thymidine or 14C-thymidine), (ii) stimulated (e.g., with an anti-CD3ε antibody, an anti-TCR antibody, a combination of an anti-CD3ε antibody and an anti-CD28 antibody, a combination of an anti-TCR antibody and an anti-CD28 antibody, 12-myristate 13-acetate (PMA), ionomycin, phytohaemagglutinin or a combination of PMA and ionomycin, (all of the afore-mentioned agents optionally in the presence of IL-2) or IL2 for a period from about 2 to about 7 days, and (iii) harvested onto glass fiber filters or directly into filter plates to measure radioactivity using a scintillation beta counter, wherein typically a control group that is not contacted with the small molecule is compared to a group that is contacted with the small molecule and the inhibition of proliferation is determined by this comparison.

In yet another preferred embodiment, the small molecule exhibits a reduction of at least about 30% of the LCK-TCR interaction in a proximity ligation assay (PLA) or in an pull down (PD) assay. It can be particularly preferred that the small molecule exhibits a reduction of at least about 50%, preferably of at least about 60%, and more preferably of at least about 70% of the LCK-TCR interaction.

A proximity ligation assay is typically carried out by (i) stimulating T cells (e.g., with an anti-CD3ε antibody, anti-TCR antibody, phytohaemagglutinin, a combination of an anti-CD3ε antibody and an anti-CD28 antibody, a combination of an anti-TCR antibody and an anti-CD28 antibody), (ii) fixing and permeabilizing the T cells, (iii) staining the fixed and permeabilized T cells with an antibody targeting any of the CD3 chains of the TCR (e.g. an anti-CD3ε antibody) and an anti-LCK antibody, wherein both antibodies are coupled to oligonucleotides that overlap in sequence such that they are amplified in a PCR reaction if they are in close molecular proximity (nm range), (iv) carrying out a PCR reaction with dye-labeled nucleotides, and (v) determining the amount of dye, wherein typically T cells that are not contacted with the small molecule are compared to T cells that are contacted with the small molecule and the reduction in interaction is determined by this comparison.

A PD assay is typically carried out by (i) stimulating T cells (e.g., with an anti-CD3ε antibody, anti-TCR antibody, phytohaemagglutinin, a combination of an anti-CD3ε antibody and an anti-CD28 antibody, a combination of an anti-TCR antibody and an anti-CD28 antibody), (ii) lysing the T cells, (iii) incubating the lysate with beads coupled to the cytoplasmic tail of CD3ε, followed by washing the beads, (iv) separating and staining the proteins bound to the beads, and (v) determining the amounts of LCK bound to the CD3ε-beads, wherein typically T cells that are not contacted with the small molecule are compared to T cells that are contacted with the small molecule and the reduction in interaction between CD3ε and LCK is determined by this comparison. The determination of the amounts is typically assessed by determining the intensity of the bands of LCK. Determining the amounts of CD3ε usually serves to control the amount of CD3ε-coupled beads used.

In an especially preferred embodiment, the small molecule exhibits an IC50 of < about 20 µM in an *in vitro* T cell proliferation inhibition assay and a reduction of at least about 30% of the LCK-TCR interaction in a PLA or an PD assay. In an even more preferred embodiment, the small molecule exhibits an IC50 of < about 10 µM in an *in vitro* T cell proliferation inhibition assay and a reduction of at least about 60% of the LCK-TCR interaction in a PLA or an PD assay.

In another preferred embodiment, the small molecule is selected from the group consisting of a compound of formula (I) 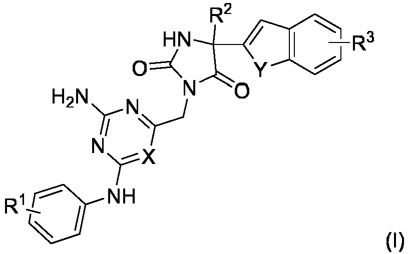 or a stereoisomer, tautomer, N-oxide, pharmaceutically acceptable salt, or hydrate thereof, wherein
- X: is N, or CH;
- Y: is O, S, or N-R^{N};
wherein R^{N} is H or C₁-C₆-alkyl; and
R¹, R² and R³ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 3- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R⁴; wherein R⁴ is halogen, C₁-C₄-alkyl, or C₁-C₄-haloalkyl;
a compound of formula (II) 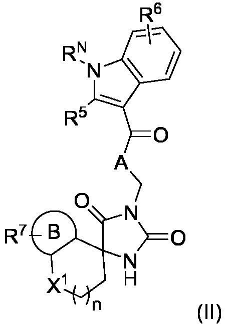
or a stereoisomer, tautomer, *N*-oxide, pharmaceutically acceptable salt, or hydrate thereof, wherein
n is 0 to 2;
A is absent or 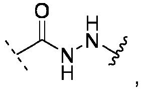 wherein the dashed line represents the connection to the methylene group and the curled line represents the connection to the carbonyl group;
ring B is a five- or six-membered monocyclic heteroaryl or a five- or six-membered monocyclic aryl;
X¹ is CH₂ or O; and
R⁵, R⁶ and R⁷ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
   or a 5- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R⁸;
   wherein R⁸ is halogen, C₁-C₄-alkyl, or C₁-C₄-haloalkyl; and
R^{N} is H or C₁-C₄-alkyl; and
a compound of formula (III) 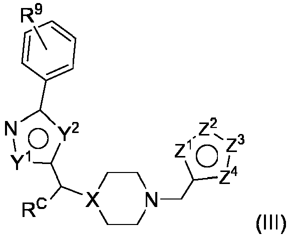
or a stereoisomer, tautomer, N-oxide, pharmaceutically acceptable salt, or hydrate thereof, wherein
   - X: is N or CR^{X};
wherein R^{X} is H or C₁₋₃-alkyl;
   and
R^{C} is H or C₁₋₃-alkyl;
or R^{X} and R^{C} together form cyclopropyl; and
Y¹ and Y² are independently selected from N or O,
   provided that not both Y¹ and Y² are N; and
   provided not both Y¹ and Y² are O; and
Z¹, Z², Z³ and Z⁴ are independently selected from N, CR^{R} or O;
   provided that not two Z¹-Z⁴ are N; and
   provided that not more than one of Z¹-Z⁴ is O and at least one of Z¹-Z⁴ is CR^{R};
   wherein R^{R} is C₁₋₆-alkyl, furanyl, phenyl, or benzyl,
   wherein both phenyl or benzyl are optionally substituted with R¹⁰; and
R⁹ and R¹⁰ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 3- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R¹¹; wherein R¹¹ is halogen, C₁-C₄-alkyl, or C₁-C₄-haloalkyl.

In a preferred embodiment, the small molecule is selected from the group consisting of

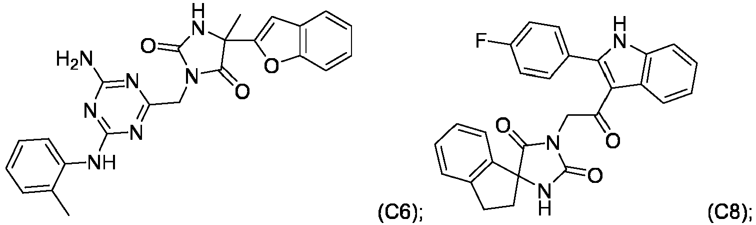

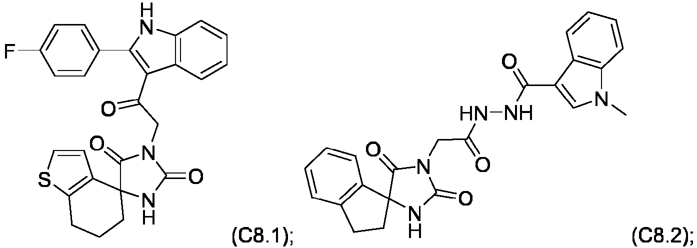

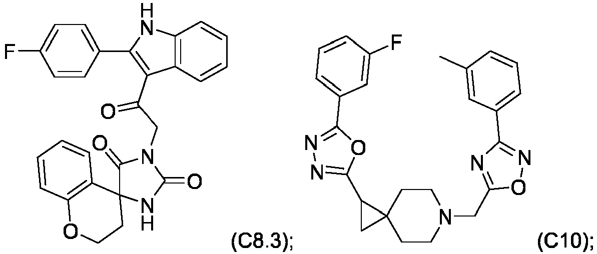

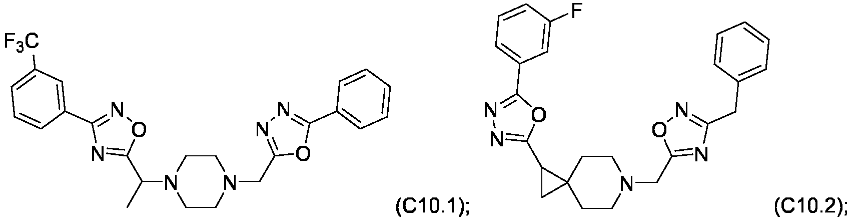

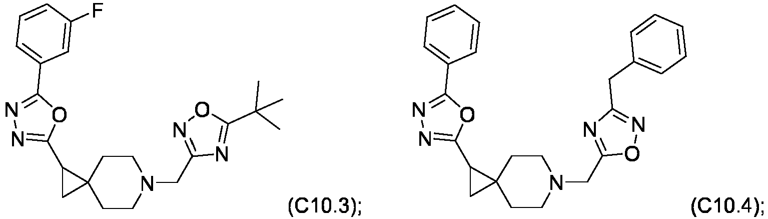

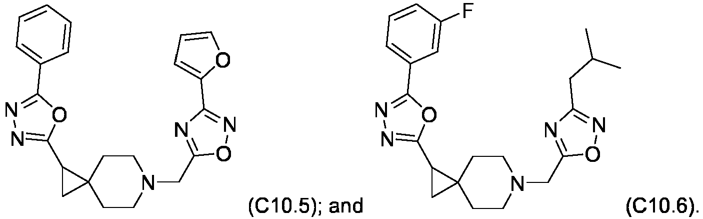

In a particularly preferred embodiment, the small molecule is selected from the group consisting of

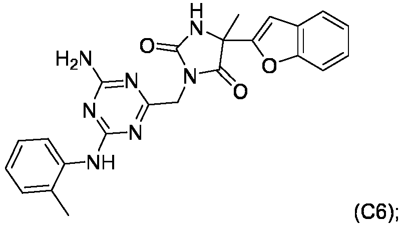

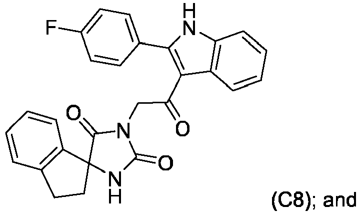

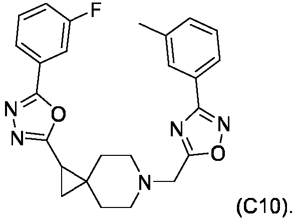

In a most preferred embodiment the small molecule is

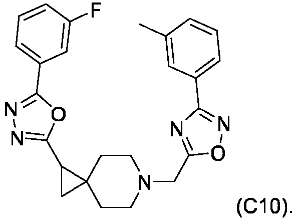

In a **sixth aspect,** the present application provides a compound of formula (I) 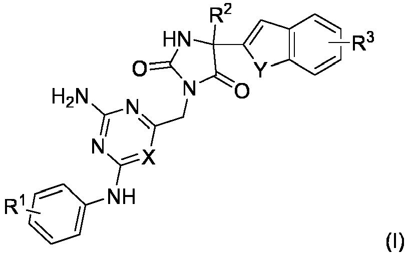 or a stereoisomer, tautomer, N-oxide, pharmaceutically acceptable salt, or hydrate thereof, wherein
- X: is N, or CH;
- Y: is O, S, or N-R^{N};
wherein R^{N} is H or C₁-C₆-alkyl; and
R¹, R² and R³ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 3- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R⁴; wherein R⁴ is halogen, C₁-C₄-alkyl, or C₁-C₄-haloalkyl.

In a preferred embodiment of the sixth aspect, the compound of formula (I) is C6

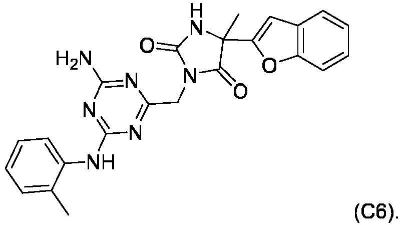

In a **seventh aspect,** the present application provides a compound of formula (II) 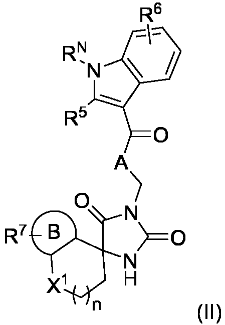 or a stereoisomer, tautomer, *N*-oxide, pharmaceutically acceptable salt, or hydrate thereof, wherein
n is 0 to 2;
A is absent or 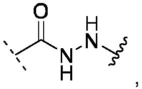
   wherein the dashed line represents the connection to the methylene group and the curled line represents the connection to the carbonyl group;
   ring B is a five- or six-membered monocyclic heteroaryl or a five- or six-membered monocyclic aryl;
X¹ is CH₂ or O; and
R⁵, R⁶ and R⁷ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
   or a 5- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R⁸;
   wherein R⁸ is halogen, C₁-C₄-alkyl, or C₁-C₄-haloalkyl; and
R^{N} is H or C₁-C₄-alkyl.

In a preferred embodiment of the seventh aspect, the compound of formula (II) is selected from the group consisting of

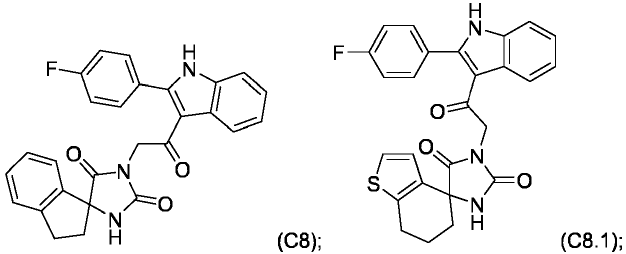

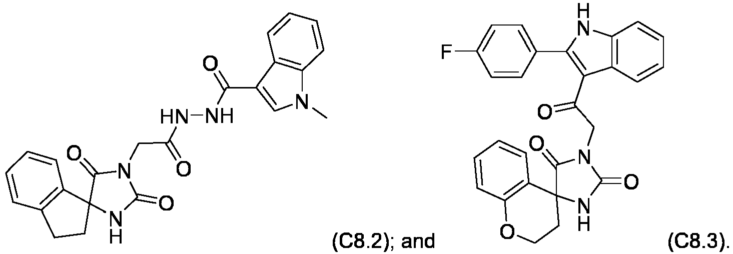

In a most preferred embodiment of the seventh aspect, the compound of formula (II) is C8

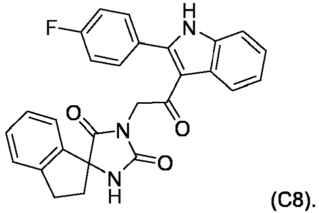

In an **eighth aspect,** the present application provides a compound of formula (III) 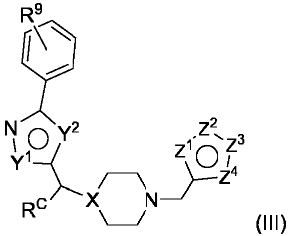 or a stereoisomer, tautomer, N-oxide, pharmaceutically acceptable salt, or hydrate thereof, wherein
- X: is N or CR^{X};

wherein R^{X} is H or C₁₋₃-alkyl;
   and
R^{C} is H or C₁₋₃-alkyl;
or R^{X} and R^{C} together form cyclopropyl; and
Y¹ and Y² are independently selected from N or O,
   provided that not both Y¹ and Y² are N; and
   provided not both Y¹ and Y² are O; and
Z¹, Z², Z³ and Z⁴ are independently selected from N, CR^{R} or O;
   provided that not two Z¹-Z⁴ are N; and
   provided that not more than one of Z¹-Z⁴ is O and at least one of Z¹-Z⁴ is CR^{R};
   wherein R^{R} is C₁₋₆-alkyl, furanyl, phenyl, or benzyl,
   wherein both phenyl or benzyl are optionally substituted with R¹⁰; and
R⁹ and R¹⁰ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 3- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R¹¹; wherein R¹¹ is halogen, C₁-C₄-alkyl, or C₁-C₄-haloalkyl.

In a preferred embodiment of the eighth aspect, the compound of formula (III) is selected from the group consisting of: 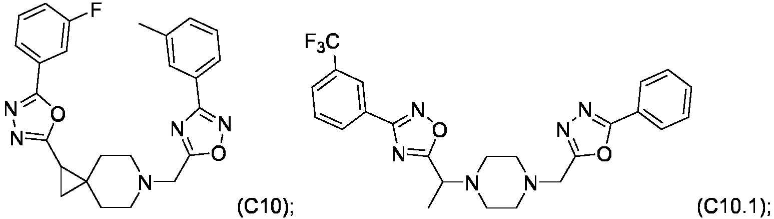 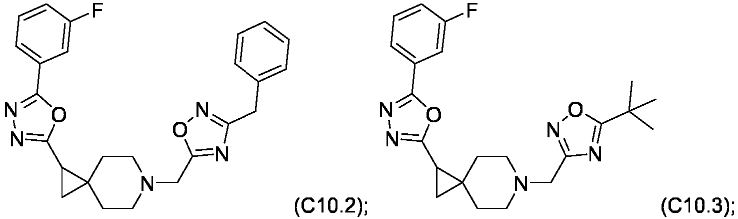 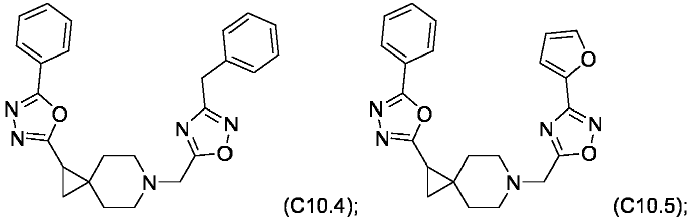 and

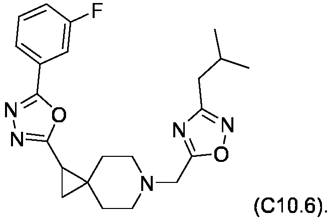

In a most preferred embodiment of the eighth aspect, the compound of formula (III) is C10

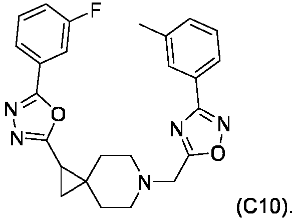

In a **ninth aspect,** the present application provides a pharmaceutical composition comprising a compound of any of the sixth, seventh or eighth aspect.

In a **tenth aspect,** the present application provides the compound of any of the sixth, seventh or eighth aspect or the pharmaceutical composition according to the ninth aspect for use in treating an autoimmune or inflammatory disease, wherein the autoimmune or inflammatory disease is preferably selected from the group consisting of rheumatoid arthritis, psoriasis, multiple sclerosis, atherosclerosis, graft-versus-host disease, allograft rejection, asthma, type 1 diabetes mellitus, vitiligo, celiac disease, Graves' disease, Hashimoto's thyroiditis, Addison's disease, systemic lupus erythematosus, Sjögren's Syndrome, systemic sclerosis, inflammatory bowel disease including Crohn's Disease and ulcerative colitis, Lichen Planus, atopic dermatitis, autoimmune hemolytic anemia, immune thrombocytopenic purpura (ITP), alopecia areata, autoimmune uveitis, sarcoidosis and ankylosing spondylitis.

In an **eleventh aspect,** the present application provides the compound of any of the sixth, seventh or eighth aspect or the pharmaceutical composition according to the ninth aspect for use in treating a side effect associated with a T cell-based immunotherapy, wherein the side effect is preferably selected from cytokine release syndrome, neurotoxicity and graft-versus-host disease.

The respective embodiments of the third aspect equally apply to the eleventh aspect.

In a **twelfth aspect,** the present application provides an *in vitro* method of manufacturing a T cell comprising a chimeric antigen receptor (CAR) or a TCR-based chimeric receptor, comprising the step of contacting the T cell with a compound of any of the sixth, seventh or eighth aspect.

The respective embodiments of the fourth aspect equally apply to the twelfth aspect.

In a **thirteenth aspect,** the present application provides a use of the compound of any of the sixth, seventh or eighth aspect to prevent or reduce early exhaustion of a T cell, optionally a T cell comprising a chimeric antigen receptor (CAR) or a TCR-based chimeric receptor.

The respective embodiments of the fifth aspect equally apply to the thirteenth aspect.

### BRIEF DESCRIPTION OF THE FIGURES

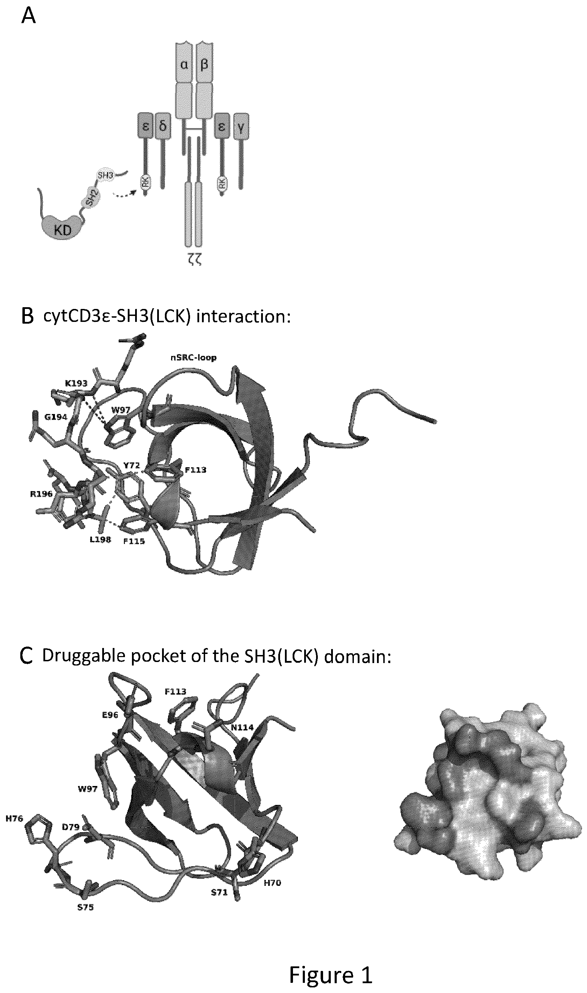

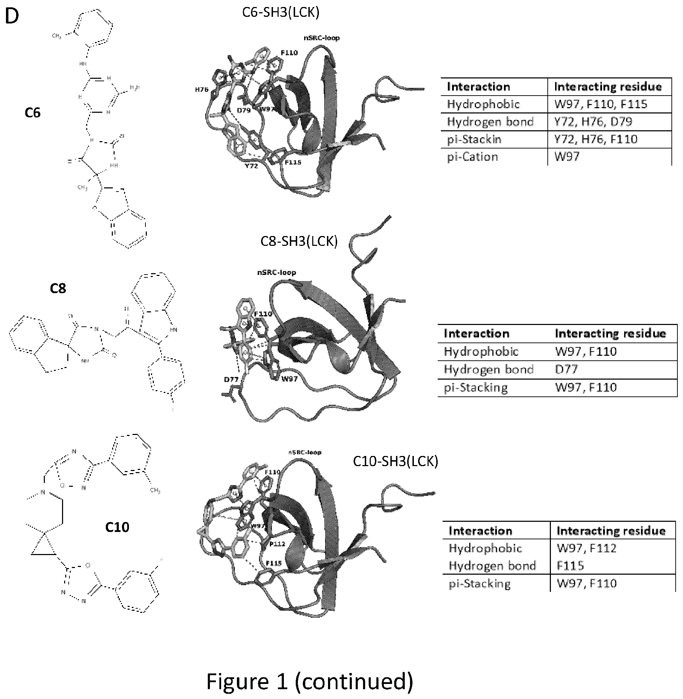

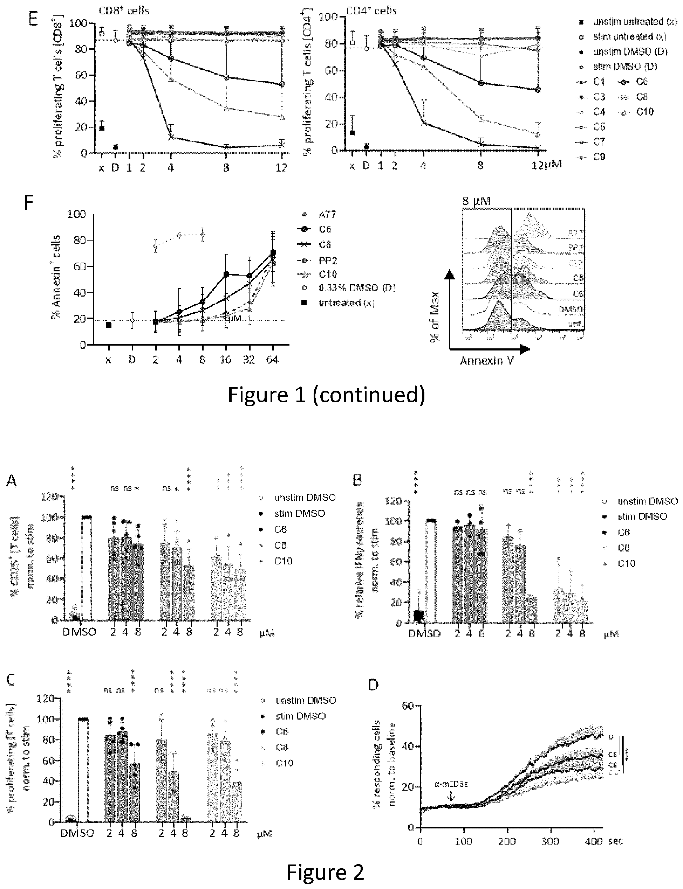

**Figure 1****: Identification of three potent small molecule inhibitors of T cell activation. (A)** The kinase LCK is recruited via its SH3 domain to the RK motif of CD3ε to induce TCR signaling. **(B)** *In silico* model of the interaction of CD3ε with the SH3(LCK) domain. **(C)** Computational modeling identified a druggable pocked within the SH3 domain of LCK. This pocked was used for the docking simulation with different small molecule compounds. **(D)** Chemical structures of the compounds C6, C8 and C10 and their predicted interactions with the SH3(LCK) domain. **(E)** Primary human T cells were either left unstimulated or stimulated with 5 µg/ml anti-hCD3ε in the presence of 0.33% DMSO (D) or the small molecules dissolved in DMSO for 96 h. The percentage of proliferating CD8⁺ or CD4⁺ T cells was assessed by flow cytometry. n= 4-10 healthy donors (HD). Means and ± SD are shown. **(F)** Primary human T cells were either left untreated (x) or treated with 0.33% DMSO or different concentrations of C6, C8, C10, PP2, A77 (PP2 and A77 are inhibitors of the kinase-domain of kinases of the Src-kinase family, including LCK). Cells were stained with Annexin V and the percentage of living cells was assessed after 24 h by flow cytometry (left). n= 3-8 HD. Means and ± SD are shown. Dotted line represents baseline of dead cells induced by DMSO treatment to visualize specific toxicity of the compounds. Representative histograms from one HD are shown (right).

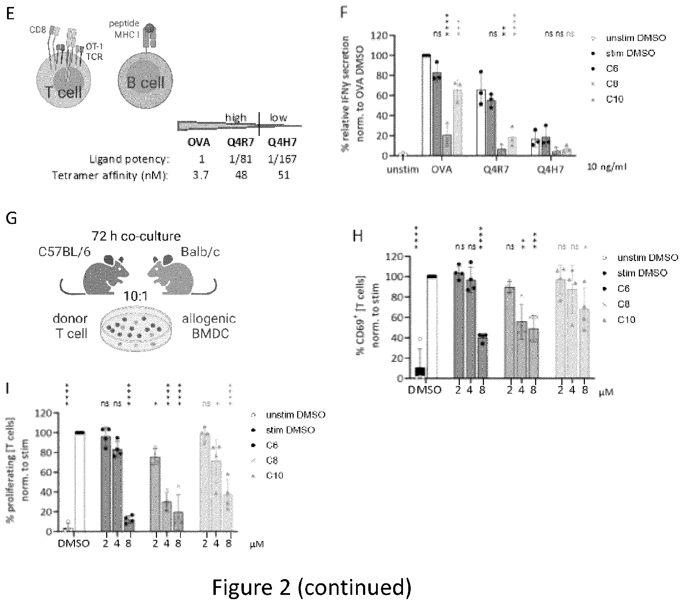

**Figure 2****: TCR-dependent activation in primary murine T cells. (A)** Splenic T cells were either left unstimulated or stimulated with 3 µg/ml anti-mCD3ε antibody in the presence of 0.33% DMSO, C6, C8 or C10. The percentage of CD25⁺ T cells by flow cytometry and **(B)** IFNγ production by ELISA, after 48 h of stimulation. **(C)** The percentage of proliferating T cells was assessed after 72 h by flow cytometry. n= 3-5 mice. **(D)** Flow cytometry analysis of calcium influx in murine splenic T cells upon stimulation with 3 µg/ml anti-mCD3ε after 60 s of baseline measurement. Cells were treated with 0.33% DMSO or 8 µM of C6, C8 or C10. n= 4 mice. Means and +SEM are shown. **(E)** OT-1 splenocytes were co-cultured with APCs derived from CD3ε^{-/-} mice, loaded with 10 ng/ml of different OVA peptide derivatives. OVAp (SIINFEKL) and Q4R7 (SIIQFERL) are high and mid affinity ligands, while Q4H7 (SIIQFEHL) is a low affinity ligand. **(F)** Cells were incubated with 0.33% DMSO or 8 µM C6, C8 or C10. IFNγ production was assessed after 48 h by ELISA. n=3 mice. All data points are normalized to the OVA stimulated DMSO control. Statistical analysis was performed using Two-way ANOVA. **(G)** Splenic donor T cells from C57BL/6 mice are co-cultured with allogenic bone marrow-derived dendritic cells (BMDC) from Balb/c mice in a cell ratio of 10:1 in the presence of 0.33% DMSO or C6, C8 or C10. **(H)** The percentage of CD69⁺ and of (I) proliferating T cells was assessed after 72 h by flow cytometry. n= 4 mice. All data points are normalized to the stimulated DMSO control. Means and ± SD are shown (except D). Each dot represents one mouse. Statistical analysis was performed using One-way ANOVA (except F). ns = not significant, *P < 0.05, **P < 0.01, ***P < 0.001, ****P < 0.0001.

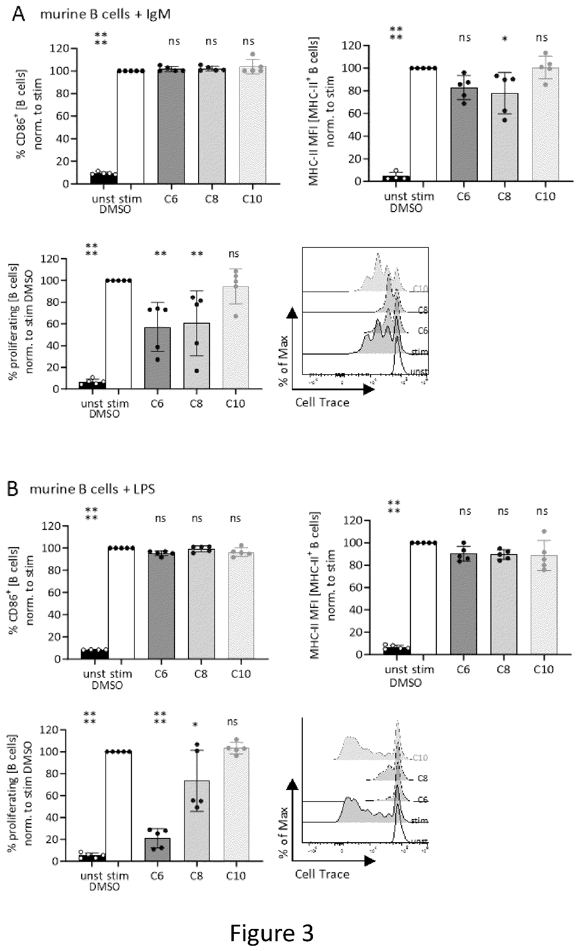

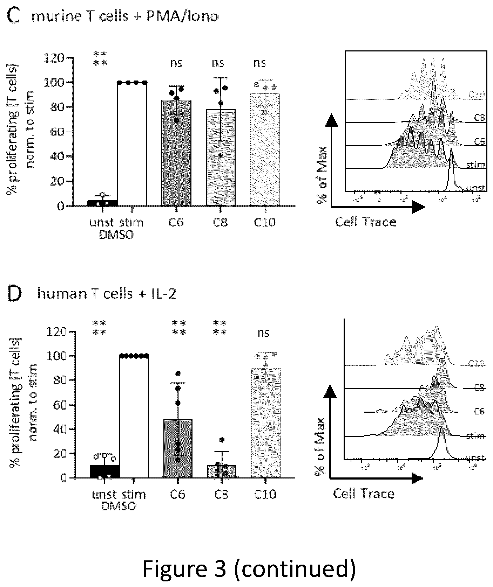

**Figure 3****: B cell activation and TCR-independent T cell activation.** Cells were incubated with 0.33% DMSO or 8 µM C6, C8, C10. **(A)** Murine splenic B were either left unstimulated or stimulated with 10 µg/ml anti-IgM(Fab'2) antibody and 5 ng/ml IL-4 or **(B)** 2.5 µg/ml LPS. The percentage of CD86⁺ B cells and the mean fluorescence intensity (MFI) of MHCII in MHCII⁺ B cells was assessed after 24 h, while proliferation was assessed after 72 h by flow cytometry. n=4 mice. **(C)** Murine splenic T cells were either left unstimulated or stimulated with 10 ng/ml PMA and 250 ng/ml ionomycin and proliferation was assessed after 72 h by flow cytometry. n=4 mice. **(D)** Primary human T cells were either left unstimulated or stimulated with 100 ng/ml IL-2 and proliferation was assessed after 96 h by flow cytometry. n=6 HD. Means ± SD are shown. Statistical analysis was performed using One-way ANOVA. ns = not significant, **P < 0.01, ***P < 0.001, ****P < 0.0001.

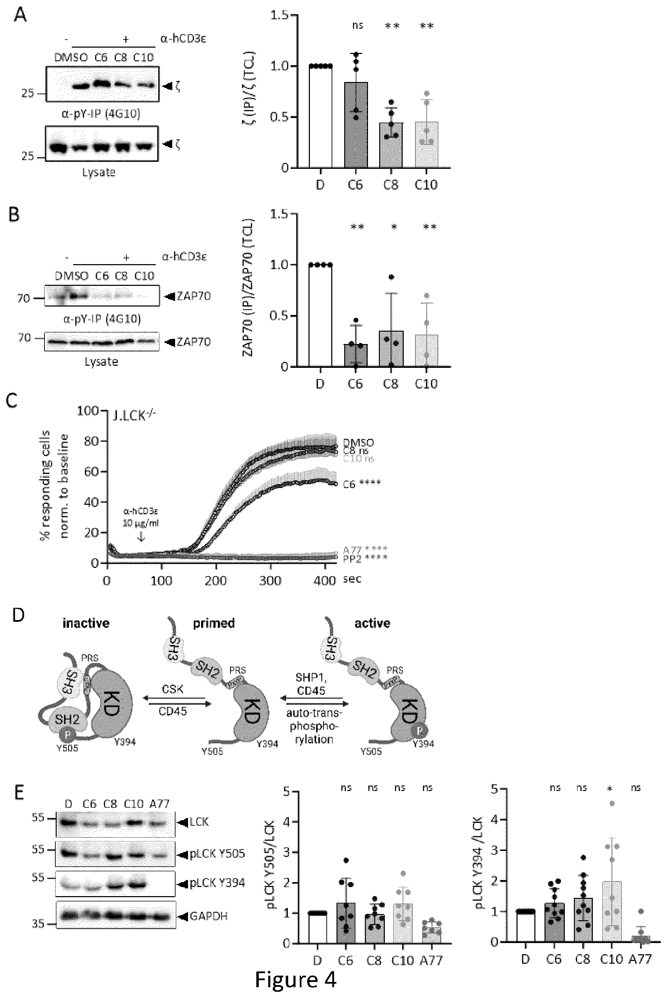

**Figure 4****: The compounds reduce early TCR signaling in Jurkat cells. (A,B)** Jurkat (JK) T cells were incubated with 0.33% DMSO or 8 µM C6, C8 or C10. Cells were left unstimulated or stimulated with 5 µg/ml anti-hCD3 antibody for 5 min at 37 °C. Then an anti-phosphoY (4G10) immunoprecipitation (IP) was performed and total cellular lysate was loaded as control. **(C)** Jurkat T cells lacking LCK (J.LCK^{-/-}) were stimulated with 10 µg/ml anti-hCD3 antibody, after measuring the baseline for 60 sec, in the presence of 0.33% DMSO or 8 µM of C6, C8, C10, PP2 or A77. Percentage of Ca²⁺ fluxing cells over time is shown. n= 4-5 independent experiments. Means with + SEM are shown. **(D)** Schematics of the regulation of LCK activity through conformational changes, phosphorylation and intramolecular interactions. **(E)** JK cells were incubated with 0.33% DMSO or 8 µM C6, C8 or C10. Basal LCK phosphorylation at tyrosine residue Y505 and Y394 was assessed by immunoblotting. Protein levels were quantified by normalizing to the DMSO control and then to the GST or total cellular lysate loading control. Each dot represents one independent experiment. Means and ± SD are indicated. Statistical analysis was performed using One-way ANOVA. ns = not significant, *P < 0.05, **P < 0.01, ****P < 0.0001.

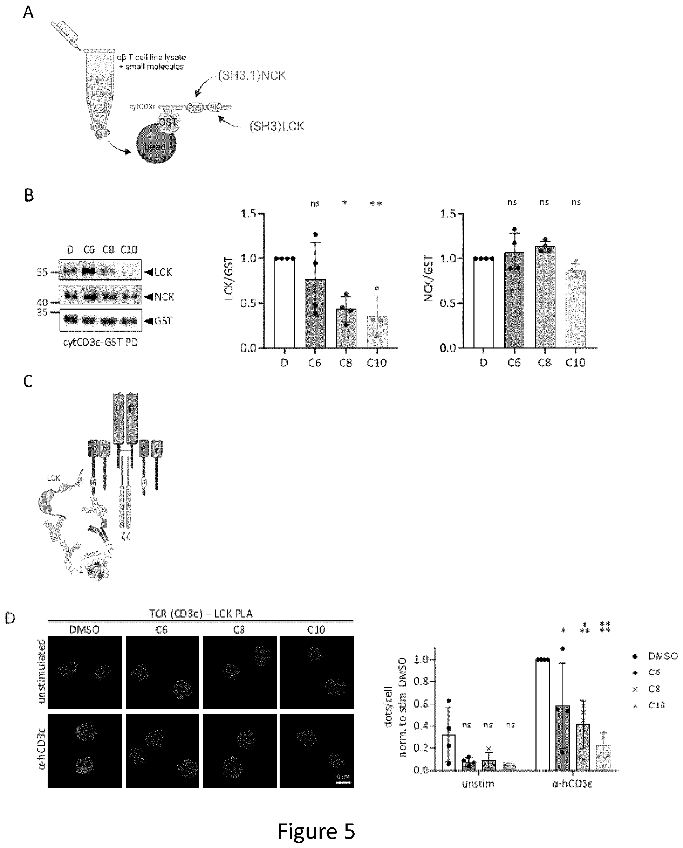

**Figure 5****: The compounds reduce interaction of LCK with the CD3ε. (A)** Schematics of the assay performed in (B). **(B)** GST-cytCD3ε pull down assay in JK lysate in the presence of 1% DMSO or 100 µM C6, C8 or C10. Immunoblotting was perfomed using an anti-LCK, anti-NCK and anti-GST antibodies. Band intensity was normalized to GST as loading control and the DMSO control for each experiment. Statistical analysis was performed using One-way ANOVA. **(C)** Schematic of a TCR(CD3ε)-LCK proximity ligation assay (PLA). **(D)** JK cells were either left unstimulated or stimulated with 5 µg/ml anti-hCD3 antibody for 5 min at 37 °C. PLA was performed between the TCR (CD3ε) and LCK. A red dot indicates a proximity closer than 80 nm. Images from one representative experiment are shown. Cell nuclei are stained with DAPI. Quantification of four independent PLA experiments. Dots per cell are normalized to the stimulated DMSO treated sample for each experiment. Statistical analysis was performed using Two-way ANOVA.

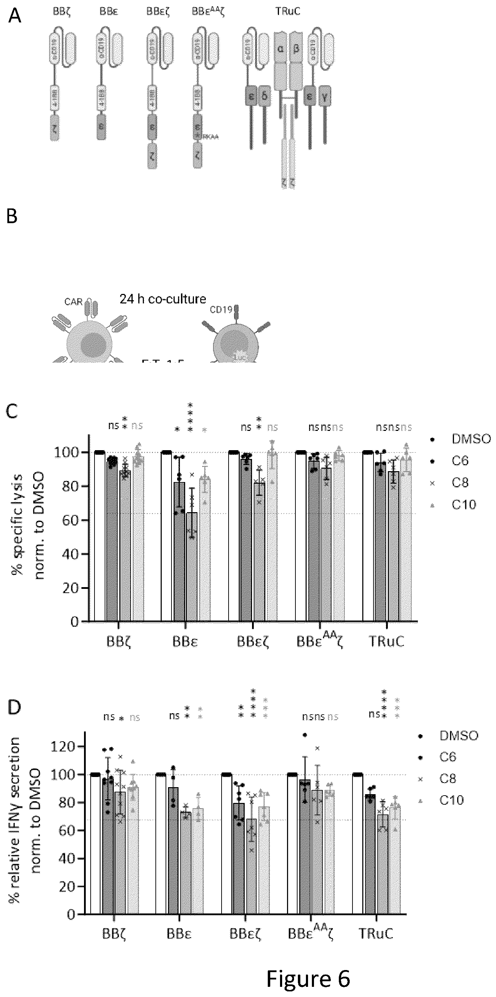

**Figure 6****: Modulation of cytotoxicity and IFNγ secretion in primary human CAR and TRuC T cells. (A)** Schematic depiction of the anti-CD19 CARs and TRuC constructs used in this study. **(B)** Primary human T cells lentivirally transduced with the respective CAR or TRuC construct are co-cultured with CD19⁺, firefly luciferase-expressing Nalm6 cells in the presence of 0.33% DMSO or 8 µM of C6, C8 or C10. Effector cell to target cell ratio 1:5. **(C)** Percentage of specific killing of the target cells was assessed by measuring the Nalm6 bioluminescence signal after 24 h of co-culture. **(D)** Percentage of relative IFNγ secretion after 24 h of co-culture was assessed by ELISA. Each dot represents one independent experiment. All data points are normalized to the DMSO control of each CAR or TRuC construct to best visualize the effect of the compound on each construct. n=2-10 healthy donors each in 1-2 independent experiments. Means with ± SD are indicated. Statistical analysis was performed using One-way ANOVA. ns = not significant, *P < 0.05, **P < 0.01, ***P < 0.001, ****P < 0.0001.

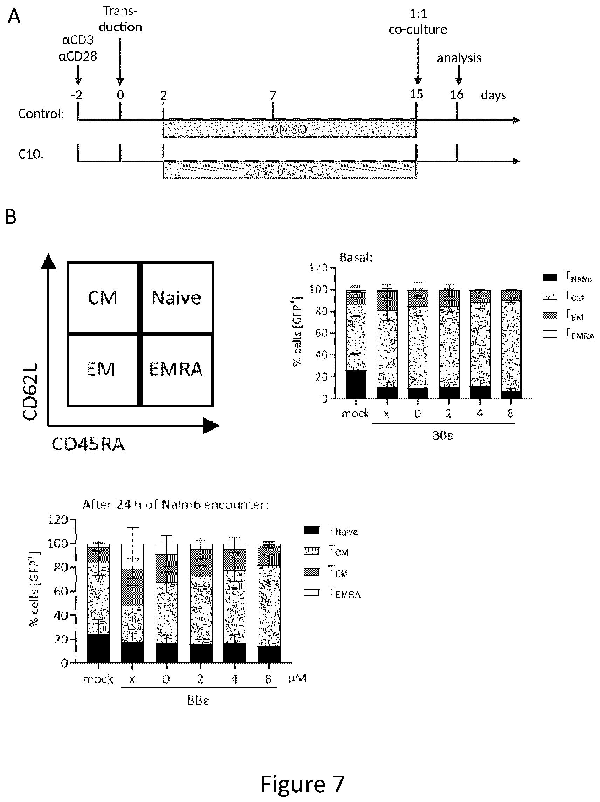

**Figure 7****: Transient rest of BB**ε **CAR-T cells with C10 promotes T_{CM}-like CAR-T cell expansion. (A)** Timeline of CAR T cell manufacturing using treatment with 2, 4 or 8 µM of C10 for a duration of 13 days. DMSO was used as control. **(B)** Phenotypic analysis of BBε CAR-T cells by flow cytometry using staining with anti-CD62L and anti-CD45RA antibodies. Percentage of respective population is shown either in basal conditions or after 24 h of co-culture with Nalm6 target cells. Each dot represents one healthy donor (HD). n=1-4 HD in 1-2 independent experiments. Means with ± SD are indicated. Statistical analysis was performed using One-way ANOVA to untreated BB ε. *P < 0.05.

### DETAILED DESCRIPTION OF THE INVENTION

### Definitions

### General definitions

As used herein, the singular form of "a" or "an" also includes the corresponding plural unless the context clearly dictates otherwise.

The term "about" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising". If hereinafter a group is defined to comprise at least a certain number of embodiments, this is also meant to encompass a group which preferably consists of these embodiments only.

The term "small molecule" as used herein refers to a small organic compound having a low molecular weight. A small molecule may be a synthetic compound not known to occur in nature or a naturally-occurring compound isolated from or known to occur in natural sources, such as e.g. cells, plants, fungi, animals and the like. A small molecule in the context of the present invention preferably has a molecular weight of less than 5000 Dalton, more preferably of less than 4000 Dalton, more preferably less than 3000 Dalton, more preferably less than 2000 Dalton or even more preferably less than 1000 Dalton. In a particularly preferred embodiment a small molecule in the context of the present invention has a molecular weight of less than 800 Dalton. In another preferred embodiment, a small molecule in the context of the present invention has a molecular weight of 50 to 3000 Dalton, preferably of 100 to 2000 Dalton, more preferably of 100 to 1500 Dalton and even more preferably of 100 to 1000 Dalton. Most preferably, a small molecule in the context of the present invention has a molecular weight of 100 to 800 Dalton. It can be preferred that a small molecule in the context of the present invention meets the "Rule of Five" as set out below and is thus orally active (i.e. has a good oral bioavailability). These rules are as follows: (i) the small molecule has no more than five hydrogen bond donors (e.g. nitrogen or oxygen atoms with one or more hydrogen atoms); (ii) the small molecule has not more than ten hydrogen bond acceptors (e.g. nitrogen or oxygen atoms); (iii) the small molecule has a molecular mass of less than 500 Dalton; (iv) the small molecule has an octanol-water partition coefficient log P not greater than 5.

The term "treating", "treat", or "treatment" as used herein embraces any form of treatment, including reversing, relieving, alleviating, or slowing the progression of the disease or any tissue damage associated with one or more symptoms of the disease. The term "treatment" is to be understood as also including the option of "prophylaxis". Thus, whenever reference is made herein to a "treatment" or "treating", this is to be understood as "treatment and/or prophylaxis" or "treating and/or preventing" unless the context clearly dictates otherwise.

The term "LCK" or "lymphocyte-specific protein tyrosine kinase" as used herein particularly refers to the protein with the UniProt ID P06239 but may include highly conserved versions of this human protein in other species, such as, e.g, mice, as well. As noted in the background of the invention section, LCK consists of an N-terminal site with a unique domain (UD), which interacts with the T cell co-receptors CD4 and CD8, a SH3 and a SH2 domains, a highly conserved kinase domain, and a short C-terminal tail.

The term "CD3ε" as used herein particularly refers to the protein with the UniProt ID P07766 but may include highly conserved versions of this human protein in other species, such as, e.g, mice, as well. As noted in the background of the invention section, LCK phosphorylates the immunoreceptor tyrosine-based activation motifs (ITAMs) of the CD3 chains. This leads to the recruitment and phosphorylation of downstream molecules and the initiation of TCR signaling.

The term "reduces" as used herein means that a given parameter, such as, e.g., an interaction between two proteins, is not at 100% but at a value below 100% and above 0. Thus, if, e.g., an interaction between two proteins is concerned, these two proteins interact at a certain level, which is set to 100%. A "reduced" interaction means that the level is no longer at 100%, but rather at, e.g., about 90%, about 80%, about 70%, about 60%, about 50%, about 40%, about 30%, about 20% or at about 10%, or even at a percentage lower than 10%.

The term "prevents" as used herein means that a given parameter, such as, e.g., an interaction between two proteins, is no longer fulfilled. Thus, if, e.g., an interaction between two proteins is concerned, these two proteins interact at a certain level. A "prevented" interaction means that these two proteins no longer interact. Thus, compared to a "reduction", a "prevention" is stronger in that it abolishes a given parameter vs. a reduction diminishes but not completely abrogates a given parameter.

The term "a T cell involving autoimmune or inflammatory disease" as used herein means that the respective autoimmune or inflammatory disease is at least to some extent influenced by T cells or, in other words, involves T cells in disease pathogenesis. If an autoimmune or inflammatory disease is "T cell-driven", this means that the T cells play a primary role in disease pathogenesis. In a T cell-involving as well as T cell-driven autoimmune or inflammatory disease, T cells are (over)activated. This (over)activation can be antagonized by the small molecule and compounds of the present application, respectively, such that the corresponding diseases can be treated by the small molecule and compounds of the present application, respectively.

The term "chimeric antigen receptor" or "CAR" as used herein relates to an artificial receptor comprising a single molecule or a complex of molecules which recognizes directly or indirectly, i.e. binds to, a target structure (e.g. an antigen) on a target cell such as a cancer cell (e.g. by binding of an antigen binding domain to an antigen expressed on the surface of the target cell) or binds to a target that can lead to tumor recognition upon presence of a linker molecule and may confer specificity onto an immune effector cell such as a T cell expressing said CAR on the cell surface. Preferably, recognition of the target structure by a CAR, either directly or indirectly, results in activation of an immune effector cell expressing said CAR. A CAR may comprise one or more protein units said protein units comprising one or more domains. The term "CAR" does not include natural receptors such as the T cell receptors.

The term "T cell receptor" or "TCR" as used herein means an endogenous or engineered T cell receptor that allows the cell expressing the same to recognize a target cell expressing the peptide-MHC recognized by the T cell receptor. The term "TCR-based chimeric receptor" as used herein refers to constructs that are engineered to target specific antigens (in particular cancer antigens). Such chimeric receptors are *inter alia* a TCR fusion construct containing an antigen-targeted single-chain variable fragment (scFv) or a single domain binder that is directly linked to the TCR via any of the ectodomains of the TCR (TRuC), an antibody-derived Fab fragment containing construct, wherein the fragment is directly attached to the constant regions of the TCRγδ chains (AbTCR), a synthetic TCR as antigen receptor having the V regions of an antibody that replaces the V regions of TCRα and TCRβ (STAR), an HLA-independent T cell receptor having the V regions of an antibody that replaces the V regions of TCRα and TCRβ (HIT), and a T cell antigen coupler, wherein an scFv is fused to an anti-CD3 scFc and the cytoplasmic tail of CD4 (TAC). Further details can, e.g., be found in Minguet et al. 2025, in particular in Figure 3 therein.

The term "T cell-based immunotherapy" as used herein means providing a patient with a cell product that is capable of recognizing a diseased cell, preferably a cancer cell. T cell-based immunotherapy can be carried out with a T cell engineered to recognize the diseased cell, preferably the cancer cell, via expression of specific TCRs or chimeric antigen receptors such as CARs or a TCR-based chimeric receptor. Adoptive cell transfer therapy with CAR-engineered T cells expressing chimeric antigen receptors is, e.g., an anti-cancer therapeutic as CAR-modified T cells can be engineered to target substantially any tumor antigen. For example, patient's T cells may be genetically engineered (genetically modified) to express CARs specifically directed towards antigens on the patient's tumor cells, then infused back into the patient.

The term "early exhaustion of a T cell" as used herein means a state of T cell dysfunction that arises, e.g., during chronic infections and cancer. The state of early exhaustion is defined by poor effector function of the T cell, sustained expression of inhibitory receptors and a transcriptional state distinct from that of functional effector or memory T cells. In cancer therapy, it is assumed that the chronic exposure to tumor-associated antigens of the T cell can result in progressive loss of T cell effector functions and self-renewal capability. Additionally, it is assumed that the chronic exposure to signaling by chimeric receptors in the absence of the specific antigen also results in progressive loss of T cell effector functions and self-renewal capability.

### Chemical definitions

In connection with the various substituents defined herein, a skilled person is aware of the point of attachment to the remainder of the molecule. For example, for substituents like halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl, it is clear to the skilled person that the point of attachment to the remainder of the molecule is present at the position indicated by "-" in the following: - halogen, -CN, -C₁-C₄-alkyl, -C₁-C₄-alkoxy, -NO₂, -C₁-C₄-perfluoroalkyl, -C(=O)O-C₁-C₄-alkyl, -C₁-C₄-aminoalkyl, -S(=O)-C₁-C₄-alkyl, -S(=O)₂-C₁-C₄-alkyl, -C(=O)-C₁-C₄-alkyl, -C₁-C₆-thioalkyl.

The term "substituted", as used herein, means that a hydrogen atom bonded to a designated atom is replaced with a specified substituent, provided that the substitution results in a stable or chemically feasible compound. Unless otherwise indicated, a substituted atom may have one or more substituents, and each substituent is independently selected. The term "substitutable atom" means that attached to the atom is a hydrogen, which can be replaced with a suitable substituent. The term "substitutable", when used in reference to a designated atom, means that attached to the atom is a hydrogen, which can be replaced with a suitable substituent. When it is referred to certain atoms or moieties being substituted with "one or more" substituents, the term "one or more" is intended to cover at least one substituent, e.g., 1 to 10 substituents, preferably 1, 2, 3, 4, or 5 substituents, more preferably 1, 2, or 3 substituents, most preferably 1, or 2 substituents. When neither the term "unsubstituted" nor "substituted" is explicitly mentioned concerning a moiety, said moiety is to be considered as unsubstituted.

The organic moieties mentioned in the above definitions of the variables are - like the term halogen - collective terms for individual listings of the individual group members. The prefix Cn-Cm indicates in each case the possible number of carbon atoms in the group.

The term "halogen" denotes in each case fluorine, bromine, chlorine or iodine, in particular fluorine, chlorine, or bromine. The term "halo" refers to fluoro, chloro, or bromo, particularly fluoro or chloro.

The term "alkyl" as used herein denotes in each case a straight-chain or branched alkyl group having usually from 1 to 6 carbon atoms, preferably 1 to 5 or 1 to 4 carbon atoms, more preferably 1 to 3 or 1 or 2 carbon atoms. Examples of an alkyl group are methyl, ethyl, n-propyl, iso-propyl, n-butyl, 2-butyl, isobutyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl1-methylpropyl, and 1-ethyl-2-methylpropyl.

The term "alkoxy" as used herein denotes in each case a straight-chain or branched alkyl group which is bonded via an oxygen atom to the remainder of the molecule and has usually from 1 to 4 carbon atoms, preferably 1 to 2 carbon atoms, more preferably 1 carbon atom. Examples of an alkoxy group are methoxy, ethoxy, n-propoxy, iso-propoxy, n-butyloxy, 2-butyloxy, iso-butyloxy, tert-butyloxy, and the like. The term "alkoxyalkyl" as used herein refers to an alkoxy group as defined herein having usually from 1 to 4 carbon atoms, preferably 1 to 2 carbon atoms, more preferably 1 carbon atom, which is bonded via an alkyl group as defined herein having usually from 1 to 4 carbon atoms, preferably 1 to 2 carbon atoms, more preferably 1 carbon atom, to the remainder of the molecule. Thus, it refers to an alkyl group, which is bonded via oxygen to a further alkyl group, which is then bonded to the remainder of the molecule. Examples of alkoxyalkyl groups are methoxymethyl, methoxyethyl, ethoxymethyl, ethoxyethyl, and the like. Alkyl and alkoxy groups can be unbranched or branched, and examples of alkyl include but are not limited to methyl, ethyl, n-propyl, i-propyl, n-butyl, i-butyl, sec-butyl, and t-butyl. Examples of alkoxy include methoxy, ethoxy, n-propoxy, i-propoxy, n-butoxy, i-butoxy, sec-butoxy, and t-butoxy.

The term "alkoxyalkyl" as used herein refers to an alkoxy group as defined herein having usually from 1 to 4 carbon atoms, preferably 1 to 2 carbon atoms, more preferably 1 carbon atom, which is bonded via an alkyl group as defined herein having usually from 1 to 4 carbon atoms, preferably 1 to 2 carbon atoms, more preferably 1 carbon atom, to the remainder of the molecule. Thus, it refers to an alkyl group, which is bonded via oxygen to a further alkyl group, which is then bonded to the remainder of the molecule. Examples of an alkoxyalkyl group are methoxymethyl, methoxyethyl, ethoxymethyl, ethoxyethyl, and the like.

The term "thioalkyl" as used herein denotes in each case a straight-chain or branched alkyl group having usually from 1 to 6 carbon atoms, preferably from 1 to 4 carbon atoms, more preferably 1 to 2 carbon atoms, and being further substituted with 1 to 5, preferably with 1 to 2 SH groups, in particular with 1 SH group. Preferably, the one SH group is terminating the straight-chain or branched alkyl group so that the SH group is bonded to an alkyl bridge, which is bonded to the remainder of the molecule.

The term "aminoalkyl" as used herein denotes in each case a straight-chain or branched alkyl group having usually from 1 to 6 carbon atoms, preferably from 1 to 4 carbon atoms, more preferably 1 to 2 carbon atoms, and being further substituted with 1 to 5, preferably with 1 to 2 amino groups, in particular with 1 amino group, wherein an amino group is a NH2 group. Preferably, the one amino group is terminating the straight-chain or branched alkyl group so that the amino group is bonded to an alkyl bridge, which is bonded to the remainder of the molecule. Examples of an aminoalkyl group are aminomethyl, aminoethyl, n-aminopropyl, 2-aminopropyl, n-aminobutyl, 2-aminobutyl, 2-amino-2-methylpropyl, and n-aminopentyl. Aminomethyl, aminoethyl, aminopropyl, and aminobutyl, are preferred, in particular aminomethyl and aminoethyl.

"=O" represents an oxo substituent.

The term "carbocyclic", "carbocyclyl", or "carbocycle" includes, unless otherwise indicated, in general a 3-to 10-membered monocyclic ring, preferably a 4- to 8-membered or a 3- to 6-membered or a 5- to 7-membered monocyclic ring, more preferably a 3-, 4-, 5- or 6-membered monocyclic ring, comprising 3 to 10, preferably 4 to 8 or 3 to 6 or 5 to 7, more preferably 3, 4, 5 or 6 carbon atoms. The carbocycle may be saturated, partially or fully unsaturated, or aromatic, wherein saturated means that only single bonds are present, and partially or fully unsaturated means that one or more double bonds may be present in suitable positions, while the Hückel rule for aromaticity is not fulfilled, whereas aromatic means that the Hückel (4n + 2) rule is fulfilled. Also "aryls" are covered by the term "carbocycles". The term "aryl" or "aromatic carbocycle" refers to aromatic carbocyclic rings based on carbon atoms as ring members, preferably 6-membered aromatic carbocyclic rings based on carbon atoms as ring members. A preferred example is phenyl. The term "carbocyclic" or "carbocyclyl", unless otherwise indicated, may therefore cover inter alia cycloalkyl, cycloalkenyl, as well as phenyl. Preferably, the term "carbocyclic" or "carbocyclyl" covers phenyl and cycloalkyl, for example phenyl, cyclopropyl, cyclobutyl, cyclopentyl, and cyclohexyl. The term "cycloalkyl" as used herein denotes in each case a monocyclic cycloaliphatic radical having usually from 3 to 10 or from 3 to 6 carbon atoms, such as cyclopropyl, cyclobutyl, cyclopentyl, cyclohexyl, cycloheptyl, cyclooctyl, cyclononyl and cyclodecyl or cyclopropyl, cyclobutyl, cyclopentyl and cyclohexyl. Cyclopropyl, cyclobutyl, cyclopentyl, and cyclohexyl are preferred.

The term "carbocyclyloxy" as used herein denotes in each case a carbocyclyl as defined herein, which is bonded via an oxygen atom to the remainder of the molecule. Examples of carbocyclyloxy include phenyloxy or cyclopropyloxy. The same applies to the terms "aryloxy" and "benzyloxy" referring to the corresponding groups, which are bonded to the remainder of the molecule via an oxygen atom.

The term "heteroaryl" or "hetaryl" or "aromatic heterocycle" or "aromatic heterocyclic ring" includes monocyclic 5- or 6-membered aromatic heterocycles, preferably 5-membered aromatic heterocycles, comprising as ring members 1, 2, 3 or 4 heteroatoms selected from N, O and S, where S-atoms as ring members may be present as S, SO or SO2, preferably as S. Examples of 5- or 6-membered aromatic heterocycles include pyridyl (also referred to as pyridinyl), i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxa-diazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl.

### Description of pharmaceutical compositions according to the present invention

The small molecule (for use) according to the present application may be comprised in a pharmaceutical composition. A pharmaceutical composition of the present application may also be designated as formulation or dosage form. A small molecule (for use) according to the present application may also be designated in the following as (pharmaceutically) active agent or active compound.

A pharmaceutical composition according to the present application may be formulated for oral, inhalative, buccal, nasal, rectal, topical, transdermal or parenteral application. Preferred non-parenteral routes include mucosal (e.g., oral, vaginal, nasal, cervical, etc.) routes, of which the oral application may be preferred. Parenteral application may be preferred and includes intravenous, intraarterial, intradermal, intrathecal, intravesical, intramuscular, epidural or subcutaneous administration, but also intranasal and inhalative administration.

Pharmaceutical compositions may be solid or liquid dosage forms or may have an intermediate, e.g. gel-like character depending inter alia on the route of administration. In general, the dosage forms can comprise various pharmaceutically acceptable excipients, which will be selected depending on which functionality is to be achieved for the dosage form. A "pharmaceutically acceptable excipient" in the meaning of the present application can be any substance used for the preparation of pharmaceutical dosage forms, including coating materials, film-forming materials, fillers, disintegrating agents, release-modifying materials, carrier materials, diluents, binding agents, and other adjuvants. Typical pharmaceutically acceptable excipients include substances like sucrose, mannitol, sorbitol, starch and starch derivatives, lactose, and lubricating agents such as magnesium stearate, disintegrants and buffering agents.

The term "carrier" denotes pharmaceutically acceptable organic or inorganic carrier substances with which the active ingredient is combined to facilitate the application. Suitable pharmaceutically acceptable carriers include, for instance, water, aqueous salt solutions, alcohols, oils, preferably vegetable oils, propylene glycol, polyoxyethelene sorbitans, polyethylene-polypropylene block co-polymers such as poloxamer 188 or poloxamer 407, polyethylene glycols such as polyethylene glycol 200, 300, 400, 600, etc., gelatin, lactose, amylose, magnesium stearate, surfactants, perfume oil, fatty acid monoglycerides, diglycerides and triglycerides, polyoxyethylated medium or long chain fatty acids such as ricinoleic acid, and polyoxyethylated fatty acid mono-, di, and triglycerides such as capric or caprilic acids, petroethral fatty acid esters, hydroxymethyl celluloses such as hydroxymethyl, hydroxyethyl, hydroxypropyl, hydroxypropyl acetate succinate, polyvinylpyrrolidone, crosspovidone and the like. Preferably, the compounds of the present invention are administered in a pharmaceutical composition comprising of lipids, interbilayer crosslinked multilamellar vesicles, biodegradeable poly(D,L-lactic-co-glycolic acid) [PLGA]-based or poly anhydride-based nanoparticles or microparticles, nanoporous particle-supported lipid bilayers and as a conjugate with an antibody.

The pharmaceutical compositions can be sterile and, if desired, mixed with auxiliary agents, like lubricants, preservatives, stabilizers, wetting agents, emulsifiers, salts for influencing osmotic pressure, buffers, colorings, flavoring and/or aromatic substances and the like which do not deleteriously react with the active compound.

If liquid dosage forms are considered for the present application, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavoring agents.

For parenteral application, particularly suitable vehicles consist of solutions, preferably oily or aqueous solutions, as well as suspensions, emulsions, or implants. Pharmaceutical formulations for parenteral administration are particularly preferred and include aqueous solutions. Additionally, suspensions may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran.

Particularly preferred dosage forms are injectable preparations. Thus, sterile injectable aqueous or oleaginous suspensions can for example be formulated according to the known art using suitable dispersing agents, wetting agents and/or suspending agents. A sterile injectable preparation can also be a sterile injectable solution or suspension in a non-toxic parenterally acceptable diluent or solvent. Among the acceptable vehicles and solvents that can be used are water and isotonic sodium chloride solution. Sterile oils are also conventionally used as solvent or suspending medium. Preferred applications for injectable preparations comprising the compounds of the present invention are intravenous, intratumoral and peritumoral administration.

Suppositories for rectal administration can be prepared by e.g. mixing the compound with a suitable nonirritating excipient such as cocoa butter, synthetic triglycerides and polyethylene glycols which are solid at room temperature but liquid at rectal temperature such that they will melt in the rectum and release the active agent from said suppositories.

For administration by inhalation, the compounds according to the present invention may be conveniently delivered in the form of an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Oral dosage forms may be liquid or solid and include e.g. tablets, troches, pills, capsules, powders, effervescent formulations, dragees and granules. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. The oral dosage forms may be formulated to ensure an immediate release or a sustained release.

A solid dosage form may comprise a film coating. For example, the dosage form may be in the form of a so-called film tablet. A capsule of the invention may be a two-piece hard gelatin capsule, a two-piece hydroxypropylmethylcellulose capsule, a two-piece capsule made of vegetable or plant-based cellulose or a two-piece capsule made of polysaccharide.

The dosage form may be formulated for topical application. Suitable pharmaceutical application forms for such an application may be a topical nasal spray, sublingual administration forms and controlled and/or sustained release skin patches. For buccal administration, the compositions may take the form of tablets or lozenges formulated in conventional manner.

The compositions may conveniently be presented in unit dosage forms and may be prepared by any of the methods well known in the art of pharmacy. The methods can include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product. Liquid dose units are vials or ampoules. Solid dose units are tablets, capsules and suppositories.

As regards human patients, the small molecule (for use) according to the present application may be administered to a patient in an amount of about 0.001 mg to about 5000 mg per day, preferably of about 0.01 mg to about 1000 mg per day, more preferably of about 0.05 mg to about 250 mg per day, which is the effective amount. The phrase "effective amount" means an amount of the small molecule (for use) according to the present application that, when administered to a mammal in need of such treatment, is sufficient to treat or prevent a particular disease or condition.

In a preferred embodiment relating to the pharmaceutical compositions of the present application, said pharmaceutical composition comprises said compound as the only pharmaceutically active agent. Alternatively, said pharmaceutical composition comprises at least one further independent pharmaceutically active agent in addition to said compound, wherein said additional active agent is typically used for the intended indication(s) as outlined above.

### Examples

### Example 1: Docking-based virtual screening against the SH3(LCK) domain identified small molecule as inhibitors of T cell proliferation

The unique nature of the interaction between the RK motif of CD3ε and the SH3(LCK) domain (Hartl et al., 2020) suggests that interfering with this protein-protein interaction bears the potential to precisely regulate TCR signaling and T cell activation (Figure 1A). Therefore, the aim was to develop small molecules as modulators targeting this interaction. To this end, a computational model of the interaction of the human SH3(LCK) domain with the cytoplasmic tail of CD3ε was generated (Figure 1B). Thereby, a druggable pocked within the SH3(LCK) domain was identified. This pocket is surrounded by residues described to mediate LCK's interaction with the RK motif (H70, S71, S75, H76, D79, E96, W97, F113 and N114) (Hartl et al., 2020) (Figure 1C). A docking-based virtual screening of more than 500.000 small molecules from the Enamine Advanced Collection database was then carried out using this SH3(LCK) pocket as target for the screening. The compounds were rated based on their predicted binding energy to SH3(LCK). Thereby, a hit list of 10 small molecules was identified (Table Ex-1), which are predicted to bind to the SH3(LCK) domain with high affinity and thus disrupt its interaction with the RK motif of CD3ε. To further characterize the pharmacokinetic properties of these 10 compounds, ADME (absorption, distribution, metabolism and excretion) parameters from their molecular structures with the SwissADME in silico web tool were predicted (Daina et al., 2017). The consensus LogPo/w (mean of five calculated partition coefficients between n-octanol and water) as measure of the lipophilicity demonstrated that all compounds are lipophilic, indicated by the positive value of the ratio. In line, the water solubility was assessed as moderate or poor. The compounds, except for C3 and C10, were not predicted to passively permeate the blood-brain barrier (BBB). The Drug-likeliness was assessed with the Lipinski filter (Lipinski et al., 2012), showing no violations against its rule-of-five, and the pattern recognition method PAINS (pan assay interference compounds) (Baell & Holloway, 2010) predicted no alerts for potentially problematic substructures.

**Table Ex-1 : Top 10 hit list of small molecules predicted to bind the druggable pocked of SH3(LCK), including their catalogue ID at the Enamine Advanced Collection database, their chemical formula, molecular weight (MW), predicted binding energy to the SH3(LCK) domain and predicted ADME parameters: Lipophilicity (Concensus Log P_{o/w}), water solubility, passive permeation of the blood-brain-barrier (BBB), number of Lipinski violations and PAINS alerts.**

| **Hit** | **Enamine Catalog ID** | **Formula** | **MW [g/mol]** | **Binding Energy [kcal/mol]** | **Consensus LogP_{o/w}** | **Water solubility** | **BBB permeant** | **Lipinski #violations** | **PAINS #alerts** |
|---|---|---|---|---|---|---|---|---|---|
| **C1** | 2229457786 | C₂₇H₂₇NO₅ | 445.5 | -7.8 | 3.51 | Moderate | No | 0 | 0 |
| **C2** | 270924377 | C₂₆H₂₇F₃N₄O₂ | 484.5 | -7.8 | 2.94 | Moderate | No | 0 | 0 |
| **C3** | 256877754 | C₂₅H₂₅N5O₂ | 427.5 | -7.7 | 4.68 | Poor | Yes | 0 | 0 |
| **C4** | 21823640784 | C₂₄H₂₂N₄O₂ | 398.5 | -7.7 | 3.82 | Poor | No | 0 | 0 |
| **C5** | 21170450081 | C₂₄H₂₀N₄O₃ | 412.4 | -7.7 | 3.8 | Poor | No | 0 | 0 |
| **C6** | Z134681002 | C₂₃H₂₁N₇O₃ | 443.5 | -7.6 | 3.61 | Moderate | No | 0 | 0 |
| **C7** | 216336862 | C₂₆H₂₀N₂O₄ | 424.4 | -7.6 | 3.99 | Poor | No | 0 | 0 |
| **C8** | Z89714290 | C₂₇H₂₀FN₃O₃ | 453.5 | -7.6 | 2.73 | Moderate | No | 0 | 0 |
| **C9** | Z1466625402 | C₂₁H₂₁FN₄O₂ | 380.4 | -7.6 | 4.33 | Poor | No | 0 | 0 |
| **C10** | Z1170443859 | C₂₅H₂₄FN₅O₂ | 445.5 | -7.6 | 4.63 | Poor | Yes | 0 | 0 |

A careful analysis of the predicted interactions of the three identified hit compounds (C6, C8 and C10) with the SH3(LCK) domain revealed that the interactions are mostly mediated by hydrophobic interactions, hydrogen bonds, non-covalent pi-stackings and electrostatic pi-cation interactions (Figure 1D).

To evaluate the effect of the identified compounds on T cell proliferation in vitro, primary human T cells were incubated either with 0.33% DMSO or the different compounds dissolved in DMSO and stimulated with an anti-hCD3 antibody. Proliferation was assessed after 96 h by flow cytometry. Three of the identified compounds, namely C6, C8 and C10, were able to inhibit proliferation in CD8+ and CD4+ T cells (Figure 1E). In contrast, C1, C3-5, C7, C9 did not affect T cell proliferation. Of note, C2 was not soluble in DMSO and was therefore not included in the experiment. The IC50 was calculated based on the proliferation assay and while the six compounds, which did not affect T cell proliferation had an IC50 over 100 µM, C6, C8 and C10 reached an IC50 of 3-19 µM (Table Ex-2).

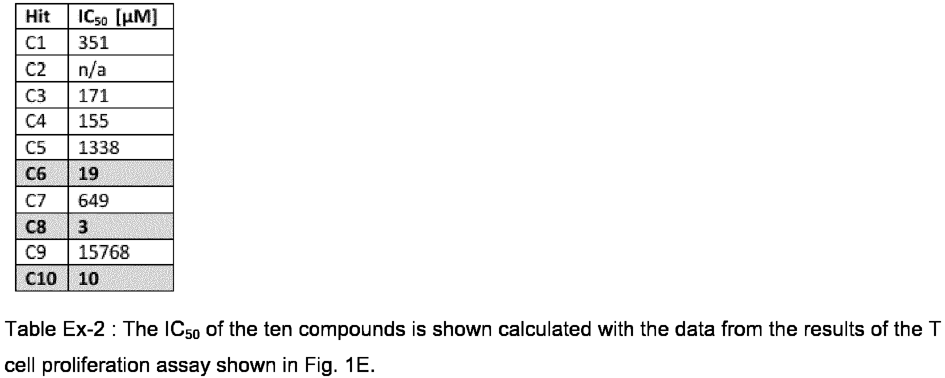

Next, the toxicity of these three compounds in comparison to the commercially available SFK inhibitor PP2 and the LCK inhibitor A-770041 (A77), which both target the kinase domain (Stachlewitz et al., 2005; Zhu et al., 1999), was assessed. Therefore, primary human T cells were left either untreated, treated with 0.33% DMSO, the compounds, PP2 or A77 for 24 h and an Annexin V staining was performed to detect apoptotic and dead cells. Treatment of the cells with 0.33% DMSO as well as treatment with up to 8 µM of C10 or PP2 did not increase the percentage of dead cells compared to the untreated control. Treatment with 8 µM of C8 increased the percentage of dead cells by 10%, and treatment with C6 by 15%, compared to the untreated control (Figure 1F). These concentrations were thus defined as suitable to be used in living cells. Treatment with 2 µM of the LCK inhibitor A77 for 24 h was toxic for the cells, resulting in 80% of dead cells, which indicates that this inhibitor can only be used for short incubation times at these concentrations.

The stability of the compounds was assessed over time at 37 °C : 8 µM of C6, C8, C10 or PP2 were added to complete medium and the medium was incubated for three or seven days at 37 °C. To test for functionality of the compounds afterwards, their effect on reducing T cell proliferation of primary human T cells was assessed, comparing the compounds incubated in medium with fresh compounds, which were kept frozen. Long incubation at 37 °C reduced the ability of the compounds to reduce T cell proliferation, however even after 1 week at 37 °C, compounds C8 and C10 were still able to reduce the percentage of proliferating T cells by 10-20% and PP2 by around 35%, indicating that C8 and C10 might have a comparable stability as the SFK inhibitor PP2.

Taken together, the docking-based virtual screening resulted in the identification of three inhibitors, which reduced T cell proliferation in a low µM range and demonstrate a similar toxicity prolife as the well-established SFK inhibitor PP2.

### Example 2: The identified compounds reduce TCR-dependent T cell activation

To further assess whether C6, C8 and C10 are able to reduce TCR-induced T cell activation in vitro, a comprehensive set of activation assays was performed in primary murine T cells.

First, splenic T cells were stimulated with an anti-mCD3 antibody to induce TCR-specific T cell activation in the presence of DMSO, C6, C8 or C10. After 48 h, the percentage of cells, which upregulated the activation marker CD25, and the amount of secreted IFNγ were assessed, while proliferation was measured after 72 h (Figure 2A-C). C6 demonstrated the mildest inhibitory effect, reducing the percentage of CD25+ cells by 20%, IFNγ secretion by 10% and proliferation by up to 40% at a concentration of 8 µM, compared to DMSO treated cells. C8 demonstrated a dose-dependent inhibition in all three assays, reducing the percentage of CD25+ cells by 40%, IFNγ by 75%, and proliferation by 90% at a concentration of 8 µM. C10 also presented a dose-dependent inhibition of CD25 upregulation and proliferation by up to 60% with 8 µM, while for IFNγ secretion already 2 µM were sufficient to reduce secretion by 60% compared to the DMSO control.

Next, intracellular calcium mobilization was investigated as a more upstream read-out for T cell activation. After 60 sec of baseline measurement, splenic T cells were stimulated with an anti-mCD3 antibody in the presence of DMSO or 8 µM of C6, C8 or C10. Incubation with C6, C8 or C10 resulted in a significant reduction of the percentage of calcium fluxing cells upon TCR stimulation by 25% (C6) to 50% (C10), compared to the DMSO control (Figure 2D).

It was then examined whether this inhibitory effect can be maintained in a more physiological setting, where the co-receptors are recruited to the TCR upon stimulation and bring co-receptor-associated LCK to promote TCR signaling. To this end, the OT-1 TCR transgenic system was used. OT-1 splenocytes were stimulated with antigen-presenting cells (APCs), derived from spleens of CD3ε^{-/-} mice, and loaded with OVA peptides or derivates exhibiting different affinities for the OT-1 TCR. The OVA (SIINFEKL) is a high affinity ligand, Q4R7 (SIIQFERL) is a mid affinity ligand, while Q4H7 (SIIQFEHL) is a low affinity ligand (Figure 2E). After 48 h of co-culture, the amount of secreted IFNγ was detected by ELISA. All compounds showed a dose-dependent reduction of IFNγ. C6 reduced IFNγ secretion by 20%, C8 by 80-90%, and C10 by 30-70%, compared to the DMSO control. These results shows that, even when co-receptor-associated LCK is involved in signal transduction, the compounds still reduce T cell activation (Figure 2F).

It was continued to assess the effect of the compounds on allogeneic T cell activation, as it occurs during allograft rejections or graft-versus-host disease (GvHD). Therefore, donor T cells were stimulated with allogenic bone marrow-derived dendritic cells (BMDC) in a ratio of 10:1 for 72 h in the presence of DMSO, C6, C8 or C10 (Figure 2G). Upregulation of the activation marker CD69 and proliferation was then assessed by flow cytometry. Incubation of the cells with 8 µM of the compounds reduced the percentage of CD69+ T cells by 40-60%, compared to the DMSO control (Figure 2H). The inhibitory effect on T cell proliferation was even stronger, demonstrating a reduction by 60-90% with 8 µM of compound (Figure 2I).

Taken together, these results demonstrate that the identified compounds are able to inhibit T cell activation induced by antibody-mediated stimulation, but also by the more physiological pMHC stimulation with co-receptor involvement. Notably, the inhibitors prevented T cell activation in allogeneic conditions, which indicates a potential therapeutic effect for allograft rejections or GvHD.

### Example 3: Compound C10 does not affect B cell activation nor TCR-independent T cell activation

To determine whether the inhibitory effects of the identified compounds are selective for T cells, murine splenic B cells were stimulated via the B cell receptor (BCR) with an anti-IgM(Fab'2) antibody or via the toll-like receptor 4 (TLR4) with lipopolysaccharide (LPS) in the presence of 8 µM of the compounds. After 24 h, the upregulation of the activation markers CD86 and MHC II was determined, and after 72 h proliferation was assessed by flow cytometry as read-out for B cell activation. Upon stimulation of the BCR, the presence of C8 reduced the MFI of MHC II by 20% and C6 and C8 reduced proliferation by 40% compared to the DMSO control. Upon stimulation with LPS, the upregulation of the activation markers were unaffected by the presence of the compounds. However, C6 reduced proliferation by 80% and C8 by 35% compared to the DMSO control, suggesting some off-target effects of C6 and C8 (Figure 3A,B). C10 did not affect the activation or proliferation of B cells, indicating a high level of T-cell specificity.

To not only assess cell specificity, but to also evaluate whether these compounds specifically target TCR-mediated signaling of T cells, murine splenic T cells were stimulated with a combination of phorbol-12-myristate 13-acetate (PMA) and ionomycin (lono). This stimulation bypasses TCR proximal signaling events by directly activating protein kinase C (PKC) and calcium influx, respectively. Treatment with 8 µM of the compounds did not significantly affect PMA/Iono-dependent T-cell proliferation (Figure 3C), but some inhibitory effect of C8 was observed compared to the DMSO control.

Taking into account that PMA/lono induce a strong T cell activation and might shield possible effects of the compounds, IL-2-dependent proliferation of primary human T cells was also investigated. IL-2 alone is a weak stimulant of T cells. C10 did not reduce IL-2-depentent T cell proliferation compared to the DMSO control, in line with the results from the B cells and the PMA/lono-mediated T cell activation, indicating a high level of specificity not only for T cells but also for TCR-mediated activation. C8 reduced the percentage of proliferating cells by 80%, demonstrating that this compound does not exclusively target the TCR-mediated signaling axis. C6 reduced IL-2-dependent T cell proliferation by 50% (Figure 3D), revealing that this compound in neither TCR nor T-cell specific.

Taken together, these results identify C10 as a promising lead compound, demonstrating a high level of specificity for T cells and also for TCR-dependent signalling.

### Example 4: Compounds C8 and C10 reduce TCR phosphorylation by targeting the kinase LCK

To mechanistically assess the inhibitory effect of the compounds on TCR signaling, Jurkat (JK) T cells were used as a model system. JK T cells were stimulated with an anti-hCD3 antibody in the presence of the compounds. An immunoprecipitation was performed with anti-phospho-tyrosine(Y) antibody-coupled beads, and after immunoblotting developed with an anti-ζ antibody to evaluate TCR phosphorylation. In contrast to C6, C8 and C10 reduced TCR phosphorylation by 50% compared to the DMSO control (Figure 4A). Next, the phosphorylation of ZAP70 was evaluated, which is a direct target of LCK activity upon TCR stimulation. Phosphorylation of ZAP70 was reduced upon treatment with all compounds compared to the DMSO control (Figure 4B), underpinning that especially compounds C8 and C10 target proximal TCR signaling.

To confirm that the reduction of TCR phosphorylation is due to an LCK-specific effect of the compounds, JK cells with a CRISPR/Cas9 KO for LCK (J.LCK-/-) were used for a calcium assay. In the absence of LCK, TCR-meditated signaling is highly compromised. To optimize the stimulation conditions to detect Ca2+ influx in the J.LCK-/- cells, the anti-hCD3 antibody was titrated in comparison to JK WT cells (data not shown). It was hypothesized that the compounds should not have any inhibitory effect on the J.LCK-/- cells. Therefore, the cells were stimulated with 10 µg/ml anti-hCD3 antibody after measuring a baseline for 60 sec in the presence of DMSO of 8 µM of compounds C6, C8 and C10 as well as the kinase inhibitors PP2 and A77 (Figure 4C). While all compounds did affect the Ca2+ response in JK WT cells (data not shown), here, C8 and C10 did not affect the percentage of Ca2+ responding J.LCK-/- cells, indicating that these two compounds indeed target the kinase LCK. In contrast, C6 still significantly reduced the percentage of responding cells by 60% compared to the DMSO control (Figure 4C), underpinning its unspecific mechanism of action. Interestedly, the supposedly specific LCK inhibitor A77 fully blocked Ca2+ influx in the J.LCK-/- cells.

To further get an idea about which domains of LCK are targeted by C8 and C10, a kinase assay was performed with human recombinant LCK in the presence of the compounds. None of the compounds affected the ability of LCK to phosphorylate the gluthathione S-transferase (GST)-fused ζ (data not shown) or CD3ε cytoplasmic tails (data not shown), excluding that the compounds affect TCR phosphorylation by affecting the kinase function of LCK. The LCK kinase inhibitor A77 was included as positive control and completely blocked phosphorylation by LCK, as expected.

Next, a possible effect of the SH3(LCK) domain was assessed. The SH3(LCK) domain is involved in the regulation of the kinase's activity through intramolecular conformational changes. In its inactive conformation, the regulatory tyrosine Y505 is phosphorylated by CSK and binding of the SH3 domain to the intramolecular proline-rich stretch (PRS) motif stabilized the closed-inactive LCK conformation (Xu et al., 1997). De-phosphorylation of Y505 and phosphorylation of Y394 result in an open and active conformation of LCK (Figure 4D). To assess whether the treatment with the compounds affects the intramolecular interaction of the SH3(LCK) domain with the PRS, the phosphorylation of Y505 and Y394 was assessed in basal conditions. Therefore, JK cells were treated with 8 µM of the compound for 1 h. Immunoblotting revealed an increase in Y394 phosphorylation after treatment with C10, indicating that C10 might interfere in the SH3(LCK) binding to the intramolecular PRS motif, and thereby promotes the active conformation of LCK (Figure 4E). However, the increase of active LCK under basal condition did not translate into increased basal TCR phosphorylation, which was unaffected by the compounds (data not shown). Furthermore, when stimulating the JK cells with an anti-hCD3 antibody the phosphorylation of Y394 and Y505 were unchanged upon treatment with the compounds (data not shown). The LCK kinase inhibitor A77 was used as positive control, blocking the LCK kinase activity.

Taken together, these results demonstrate that the novel compounds C8 and 10 target early TCR signaling indicated by reduced TCR and ZAP70 phosphorylation through targeting the kinase LCK.

### Example 5: compounds C8 and C10 target the protein-protein interaction of LCK and the TCR

To assess for the specific mechanism of action of the identified compounds and to investigate whether the compounds indeed target the interaction of the SH3(LCK) domain with CD3ε, a pull-down assay was performed with un-phosphorylated GST-cytCD3ε beads in JK lysate. The SH3(LCK) domain can bind to the RK motif. The adapter protein NCK binds via its SH3.1 domain to the PRS of CD3ε (Figure 5A). The pull-down (PD) assay was performed in the presence of DMSO, C6, C8 or C10 and immunoblotting was conducted. Binding of LCK to the GST-CD3ε beads was reduced in the presence of C8 and C10 by around 50% compared to the DMSO control (Figure 5B), in line with the 50% reduction of TCR phosphorylation seen in Figure 4A. This demonstrates that the compounds target specifically the interaction of the SH3(LCK) domain with CD3ε. Binding of the SH3.1(NCK) domain to CD3ε was unaffected by all compounds, indicating that the compounds are selective for the SH3(LCK) domain (data not shown).

To further confirm this mechanism of action, a proximity ligation assay (PLA) was performed between the TCR (CD3ε) and LCK (Figure 5C). Here, a red dot in the microscope indicated a closer proximity of 80 nm of the two proteins of interest. The JK cells were left unstimulated or stimulated with an anti-hCD3ε antibody for 5 min in the presence of DMSO or compounds C6, C8 or C10. Under basal conditions, treatment with all compounds already reduced TCR-LCK proximity, however not significantly. In stimulated cells, treatment with C6 reduced TCR-LCK proximity by 40%, treatment with C8 by 60% and treatment with C10 by 70%, compared to the DMSO control (Figure 5D), confirming the results obtained with the cytCD3ε-PD.

Taken together, these results strongly support a specific mechanism of action for C8 and C10, namely a specific targeting of the RK-(SH3)LCK interaction.

### Example 6: the compounds control the activity of primary human CAR and TRuC T cells

To investigate a potential clinical application of the identified compounds, their inhibitory effect on CAR and TRuC constructs was assessed. While CAR and TRuC T cells have shown remarkable success in the treatment of leukemia, patients also suffer from severe side effect such as cytokine release syndrome (CRS) or neurotoxicity. Therefore, specifically modulating the activity of the CAR and TRuC T cells would be a desirable therapeutic concept.

Five different CD19-targeting constructs were included in the panel. First, the FDA-approved CAR construct, containing the 4-1BB-derived co-stimulatory domain and the ζ-derived signaling domain (BBζ). The signaling capacity of this construct does not dependent on the RK-LCK interaction. Then, a CAR containing CD3ε instead of ζ (BBε), and a CAR containing a CD3ε and ζ signaling domain (BBεζ) were used. The activity of these constructs is dependent on LCK recruitment via the RK motif (Hartl et al., 2020; Velasco Cárdenas et al., 2023). Furthermore, a BBεζ CAR containing the loss-of-function mutation RKAA within the RK motif (BBεAAζ) was analyzed, and finally, a TRuC construct was included, where the single-chain variable fragment targeting CD19 is fused to CD3ε (Figure 6A).

Primary human T cells were transduced with lentiviral vectors encoding the different constructs. Then, the CAR and TRuC T cells were co-cultured with CD19-experessing Nalm6 cells to specifically engage the CARs and TRuCs (Figure 6B). After 24 h, specific tumor cell lysis was assessed by measuring bioluminescence signal of the firefly luciferase-expressing Nalm6 cells, while IFNγ secretion was assessed by ELISA. Since all constructs have a different capacity to kill tumor cells and secrete IFNγ in vitro (data not shown), the data were normalized to the DMSO control of each construct to best visualize the specific effect of the compounds on each CAR/TRuC construct.

All three compounds reduced the killing activity of the BBε CAR, which is the one that is most dependent on LCK-mediated signaling. The killing capacity of the BBεζ CAR was only reduced by C8, suggesting that the presence of ζ compensates for the inhibitory effect of the compounds. In line, the killing capacity of the TRuC was hardly affected, also suggesting some compensation by the additional CD3 chains (Figure 6C).

For the negative controls BBζ and BBεAAζ, any inhibitory effect of the compounds was not presumed due to the lack of a (functional) RK motif in these constructs. Indeed, only a minimal effect of the compounds was observed, strengthening the hypothesis that, at least C8 and C10 specifically target the LCK-RK interaction, also in the context of CAR and TRuC constructs. To rule out off-target effects on target cells, we performed luciferase-based proliferation assays with Nalm6 and other leukemia cell lines. While C8 inhibited leukemia cell proliferation, C10 had no direct effect, reinforcing its T-cell specificity (data not shown).

When evaluating IFNγ secretion, the effects of the compounds became even clearer. Especially C8 and C10 significantly reduced IFNγ secretion of the BBε CAR, the BBεζ CAR, and the TRuC, while hardly affecting the BBζ and BBεAAζ control constructs (Figure 6D).

These results demonstrate that the compounds can modulate the cytotoxicity and IFNγ secretion of RK-containing CAR and TRuC T cells, demonstrating a therapeutic potential of the compounds to treat CAR-induced side effects like CRS.

### Example 7: Compound C10 promotes T_{CM}-like CAR-T cell expansion

To investigate whether C10 treatment enhances the expansion of BBε-expressing CAR-T cells with a central memory (T_{CM})-like phenotype while reducing effector memory (T_{EM})-like characteristics, BBε CAR-T cells were cultured in the presence of 2, 4, or 8 µM C10 for 13 days, starting two days after transduction (Figure 7A).

Transduction efficacy was assessed at day 7 after transduction. C10-treated CAR T cells demonstrated similar GFP and CAR levels than DMSO-treated CAR T cells (data not shown). Expansion was progressively reduced in a dose-dependent manner compared to the DMSO control (Figure 7B). After the pretreatment with C10, the cells were washed and analyzed by flow cytometry. Phenotypic analysis revealed an increased proportion of T_{CM}-like CAR-T cells, alongside a reduction in T_{EM}- and T_{EMRA}-like populations, even in the basal state (Figure 7C). These effects were further amplified following a 24 h Nalm6 target cell encounter, with 8 µM C10 yielding a T-cell population closely resembling mock-transduced T cells. Importantly, C10 pretreatment did not alter the phenotype of BBζ CAR-T cells (data not shown), confirming its specificity for targeting CD3ε-mediated signaling. Functional assessment demonstrated that after 24 h of stimulation with Nalm6 cells, C10-pretreated CAR-T cells are effectively activated by upregulating the activation markers CD69, 4-1BB, and CD25 (data not shown). Furthermore, degranulation and cytotoxic activity against Nalm6 target cells remained comparable to DMSO-treated controls and was not affected by the prior treatment with C10 (data not shown).

These findings demonstrate that C10 functions as a pharmacological on/off switch for CAR-T-cell activity and induced a favorable expansion of central-memory-like CAR T cells, which have been linked with an enhanced anti-tumoral performance *in vivo* (López-Cantillo et al., 2022). This resting protocol therefore may have potential applications in clinical CAR-T-cell manufacturing to enhance therapeutic efficacy by inducing a favorable CAR-T-cell phenotype.

### Example 8: Modeling of additional active compounds based on the predicted interactions of C6, C8 and C10 with the SH3(LCK) domain

Based on the results obtained for compounds C6, C8 and C10 as described in the above examples and based on the predicted interactions of these three compounds with the SH3(LCK) domain (see also Figure 1 (D)), additional compounds based on the structures of the compounds C6, C8 and C10 were modelled(i.e., tested *in silico* and docketed against the druggable pocket of the SH3(LCK) domain), which are also predicted to strongly interact with the SH3(LCK) domain and which are thus also assumed to be active like their "underlying starting" compounds C6, C8 and C10.

Table Ex-3 lists C8 and C10 together with some additional compounds and *inter alia* provides their predicted binding energy towards the druggable pocket of the SH3(LCK) domain, which indicates a strong binding.

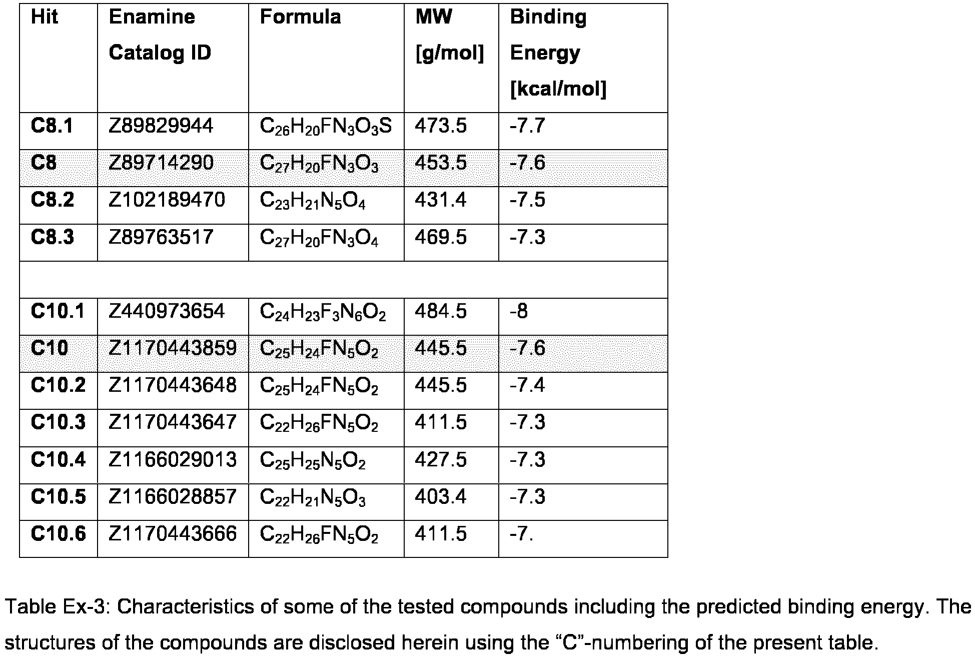

The modeling resulted in the three rather narrow Markush-structures of compounds of formula (I) [based on compound C6], formula (II) [based on compound C8], and formula (III) [based on compound C10] as disclosed in the specification and the claims of the present application.

### Example 9: Materials and Methods

**Compounds.** Tables Ex-1 and Ex-3 list compounds as used herein and provide *inter alia* an "Enamine catalog ID" for each compound. The structures of these compounds are available at Enamine under the unique catalog ID (www.enamine.net) and the compounds are commercially available at Enamine (www.enamine.net).

**CD3ε-SH3(LCK) interaction prediction.** To predict the key residues in the interaction between CD3ε and SH3(LCK), the AutoDock CrankPep tool was used (Zhang & Sanner, 2019). The tool allows the docking of flexible peptides. The docking box dimensions were 24 Å by 20 Å by 24 Å with a grid spacing of 1 Å and was centered around the previously described interaction sites between CD3ε and SH3(LCK) (Hartl et al., 2020).

**Docking and interaction characterization.** In order to identify candidates with high affinity for the SH3(LCK) domain, a High Throughput Virtual Screening (HTVS) was performed. The AutodockVina software (Allouche, 2012) was used as the docking software. The docking was centered around the druggable pocket in the region of SH3(LCK) domain that interacts with CD3ε. The docking box dimensions were 16 Å by 14 Å by 18 Å with a grid spacing of 1 Å. The Enamine Advanced Collection, a database with over 500.000 drug-like compounds was used in the screening (https://enamine.net/compound-collections/screening-collection/advanced-collection). The preparation of the compounds for docking was performed using the AutoDockTools4 package (Morris et al., 2009). For the parallelization and distribution of the docking tasks to our high performance computing cluster, the in-house developed Molecular Sniper software (Blanco et al., 2020) was used. For the best candidates, the interactions established between them and the pocket of SH3(LCK) were calculated using the Protein-Ligand Interaction Profiler (PLIP) software (Salentin et al., 2015).

**Prediction of ligands physicochemical parameters.** The physicochemical parameters of each candidate were predicted using the SwissADME web server (Daina et al., 2017). The SwissADME web tools suite is specifically designed to predict ADME properties of chemical compounds.

**CD25 upregulation and IFNγ production.** 0.2x10⁶ splenocytes were incubated at 37 °C in 96-well plates stimulated with 3 µg/ml plate-bound anti-mCD3ε antibody (145-2C11, Biolegend), or with APC derived from CD3ε^{-/-} mouse spleens and loaded with 10 ng/ml OVA, Q4R7 or Q4H7 peptide (for 3-4 h) in a ratio of 1:1 in the presence of 0.33% DMSO or C6, C8 or C10 dissolved in DMSO. After 48 h, cells were stained with anti-mCD4-PeCy7 (RM4-5, eBioscience), anti-mCD8-FITC (Invitrogen) and anti-mCD25-APC (PC61.5, eBioscience) antibodies. CD25 upregulation was assessed by flow cytometry. Alternatively, the supernatant of the stimulated cells was collected after 48 h of stimulation and IFNγ secretion was assessed using a commercial ELISA kit (eBioscience).

**T cell proliferation.** 0.5x10⁶ splenocytes were stained with 1 µM CellTrace Violet and stimulated at 37 °C in 96-well plates with 3 µg/ml plate-bound anti-mCD3ε antibody (145-2C11) or 10 ng/ml PMA and 250 ng/ml ionomycin in the presence of 0.33% DMSO or C6, C8 or C10 dissolved in DMSO. After 72 h, the cells were stained with anti-mCD4-PeCy7 (eBioscience/Biolegend) and anti-mCDB-FITC/PeCy7 (Invitrogen/Biolegend) antibodies. Alternatively, freshly thawed human peripheral blood lymphocytes were pre-activated with 2 µg/ml phytohaemagglutinin (PHG, Sigma) for 48 h in the presence of 10 ng/ml IL-2 (Peprotech) and then cultures for 5-7 days in the presence of 10 ng/ml IL-2 before used for an experiment. 0.5x10⁶ cells were stained with 1 µM CellTrace Violet and stimulated at 37 °C in 96-well plates with 3 µg/ml plate-bound anti-hCD3ε antibody (UCHT1, serum) or 100 ng/ml IL-2 in the presence of 0.33% DMSO, C1, C3-10 [1,2,4,8,12 µM] dissolved in DMSO, or 8 µM of C6, C8, C10, PP2, incubated in 10 % FCS RPMI for 3 or 7 days at 37 °C. After 96 h, the cells were stained with anti-hCD8-APC (Beckmann Coulter) and anti-hCD4-PeCy7 (Biolegend) antibodies. T cell proliferation was determined by the extent of CellTrace Violet dye (ThermoFisher) dilution measured by flow cytometry (Gallios Beckman Coulter/Attune).

**Toxicity.** 6-well plates were pre-coated with 1 µg/ml anti-hCD3ε (UCHT1) and 1 µg/ml anti-hCD28 antibody for 1 h at 37 °C. Frozen PBMCs from healthy donors were defrosted and 1x10⁶ cells/ml were plated into wells in the presence of 1000 U/ml of h-IL-2 (Preprotech). 3 days later, 0.2x10⁶ cells per sample were incubated with 0.33% DMSO or C6, C8, C10, PP2 or A77 dissolved in DMSO for 24 h. The percentage of apoptotic and dead cells was determined with Annexin-FITC staining (BD Pharmingen).

**B cell proliferation.** 0.3x10⁶ MACS purified untouched splenic B cells (Miltenyi Biotec) were stained with 1 µM CellTrace Violet and stimulated at 37 °C in 96-well plate with 10 µg/ml anti-IgM(Fab'2) antibody and 5 ng/ml IL-4 or with 2.5 µg/ml LPS in the presence of 0.33% DMSO or 8 µM of C6, C8, C10 dissolved in DMSO. Cells were stained with anti-CD19-PE antibody (Biolegend) as B cell marker. B cell activation was assessed by flow cytometry after 24 h of stimulation by staining with anti-CD86-PE (Biolegend) and anti-MCHII-APC (Biolegend) antibodies, while proliferation was determined by the extent of CellTrace Violet dye (ThermoFisher) dilution measured by flow cytometry after 72 h of incubation (Attune).

**Allogeneic T cell activation.** Splenic T cells for C57BL/6 mice were MACS purified using the Pan T cell isolation kit (Miltenyi Biotec), labeled with 1 µM CellTrace Violet (ThermoFisher) and co-cultured with LPS-stimulated, allogeneic bone marrow-derived dendritic cells (BMDCs) from Balb/c mice at a cell ratio of BMDC:T cells = 1:10 in 96-well format. The cells were treated with 0.33% DMSO or C6, C8 or C10 dissolved in DMSO and after 72 h cells were stained with anti-mCD69-PE, anti-mCD4-APC and anti-mCD8-PeCy7 antibodies (all Biolegend). T cell proliferation was determined by the extent of CellTrace Violet dye (ThermoFisher) dilution measured by flow cytometry (LSR Fortessa). T cell viability was measured using the Zombie NIR Fixable Viability Kit (BioLegend).

**Intracellular calcium mobilization.** For Ca²⁺ influx measurement, 0.5×10⁶ cells per sample were resuspended in medium containing 1% FCS and incubated for 30 min at 37 °C with 5 µg/ml of Indo-1 and 0.5 µg/ml of pluronic F-127 (Molecular Probes). After washing, the cells were kept in the dark on ice. Right before measurement, the cells were pre-warmed 5 min at 37 °C in the presence of 0.08% DMSO or 8 µM C6, C8 or C10 dissolved in DMSO. The Ca²⁺ response was induced by addition 3 µg/ml anti-mCD3ε (145-2C11, Biolegend) or 10 or 0.5 µg/ml anti-hCD3ε (UCHT1, serum) antibody after 60 s of baseline measurement. The change of the ratio Indo-bound versus Indo-unbound was measured with a MaxQuantX Flow Cytometer (Miltenyi Biotech). Data were normalized to the baseline with FlowJo software version 9.3.2.

**Kinase assay.** 2 µg hcytCD3ε-GST or hcytζ-GST and 17 µM ATP were incubated in 1x kinase buffer (40 mM Hepes, 10 mM MgCl₂, 3 mM MnCl₂) in the presence or absence of 50 ng LCK-GST (ENZO; BML-SE356-005) with 1% DMSO or 100 µM C6, C8, C10 or A77 dissolved in DMSO for 15 min at 30 °C. Reaction was stopped by adding 5x non-reducing sample buffer and boiling the sample for 5 min at 95 °C.

**Cell stimulation for immunoblotting.** 3x10⁶ Jurkat cells per sample were incubated with 0.33% DMSO or 8 µM C6, C8, C10 or A77 dissolved in DMSO for 1 h at 37 °C. Afterwards, cells were left unstimulated or stimulated with 5 µg/ml anti-hCD3ε (UCHT1, serum) antibody, and then lysed in 80 µl EMBO lysis buffer (20 mM Tris-HCl (pH 8), 137 mM NaCl, 2 mM EDTA, 10% glycerol, protease inhibitor cocktail (Sigma), 1 mM PMSF, 5 mM iodoacetamide, 0.5 mM sodium orthovanadate, 1 mM NaF and 0.3% Brij96V) for 30 min at 4 °C. Samples were stored at -20 °C with 5x non-reducing sample buffer until further use.

**Immunoprecipitation (IP) and pull down (PD).** For the IP, 60x10⁶ Jurkat (JK) cells were treated with 0.33% DMSO or 8 µM of C6, C8, C10 dissolved in DMSO and incubated for 1 h at 37 °C. Cells were either left unstimulated or stimulated with 5 µg/ml anti-hCD3ε antibody (UCHT1, serum) for 5 min at 37 °C and then lysed in 1 ml EMBO lysis buffer (see above) for 1 h at 4 °C. 10 µl of anti-pY antibody 4G10-coupled agarose beads (Merck Millipore) were added to 900 µl of the lysate and incubated over night at 4 °C. For the PD, 55×10⁶ JK cells were resuspended in 1 ml of EMBO lysis buffer and lysed for 1 h at 4 °C. 10 µl of cytCD3ε-GST-coupled sepharose beads were incubated with the lysate overnight in the presence of 1% DMSO, or 100 µM of C6, C8 or C10 dissolved in DMSO. In all cases, beads were washed 3x with 500 µl EMBO washing buffer (20 mM Tris-HCl (pH 8), 137 mM NaCl, 2 mM EDTA, 10% glycerol, 0.3% Brij96V) and stored with 20 µl 5x non-reducing sample buffer at -20 °C until further use.

**SDS PAGE and Immunoblotting.** Samples were loaded on 12% SDS gels and subjected to SDS-PAGE separation and transferred to PVDF membranes for immunoblotting by semi-dry transfer (18 V; 1 h). Quantification of the band intensities was performed with the ImageQuantTL software (GE Healthcare) after chemoluminescence detection. Band intensity was set to 1 for the DMSO control.

### In situ proximity ligation assay (PLA)

1x10⁵ cells per sample were starved in 0% FCS RPMI and rested on diagnostic microscope slides (Thermo Fisher Scientific) for 1 h at 37 °C. Cells were left unstimulated or stimulated with 5 µg/ml anti-CD3ε (UCHT1) for 5 min at 37 °C. Cells were then fixed in 2% PFA for 15 min, permeabilized with 0.5% saponin for 30 min and blocked. Blocked cells were stained according to the manufacturer's instructions with the Duolink kit (Olink Bioscience) with goat anti-CD3ε (Everest Biotechnology) and mouse anti-LCK (3A5, Santa Cruz Biotechnology). Nuclei were stained with DAPI (Roth). A total of 5-7 images (on average 700 cells) per sample were taken at ×60 with a confocal microscope (Nikon C2) and analyzed with BlobFinder (Woessner et al., 2024).

**Generation of lentiviruses and lentiviral transduction.** 10⁷ HEK293T cells were plated on a 15 cm plate in 20 ml of DMEM and incubated at 37 °C and 7.5% CO₂. After 24 h, the medium was changed, and the cells were transfected with the respective constructs and the packaging plasmids pMD2.G (envelope) and pCMVR8.74 (gag/pol) using PEI transfection. The virus-containing supernatant was collected 24 and 48 h after transfection and was concentrated with a 10% sucrose gradient (supplemented with 0.5 mM EDTA) centrifugation for 4 h at 10,000 xg and 6 °C. After centrifugation, supernatant was discarded and the virus pellet was resuspended in 100 µl of PBS and stored at -80 °C.

**Primary human T cell activation, transduction and expansion.** Isolated peripheral blood mononuclear cells (PBMCs) from healthy donors (ethics approval no. 22-1275-S1) were thawed and resuspended in medium supplemented with 500 U/ml recombinant human IL-2 (PeproTech) and activated with 1 µg/ml anti-hCD3 and anti-hCD28 antibodies. At 48-72 h, the remaining PBMCs were mostly T cells (>99 %). Primary human T cells were then lentivirally transduced using spin infection in the presence of 5 µg/ml protamine sulfate (Sigma) and 500 U/ml IL-2 with a multiplicity of infection (MOI) of 4. Transduced T cells were checked for CAR/TRuC expression 5-7 days after transduction using a biotinylated primary goat anti-mouse F(ab')2 (Invitrogen) antibody followed by streptavidin-APC (BioLegend). Cells were cultured in medium supplemented with 100 U/ml IL-2 for a maximum of 7 d after transduction before use for the indicated experiments.

**CAR and TRuC T cell killing assay and IFNγ production.** For the Bioluminescence-based cytotoxicity assay, luciferase-expressing tumor cells (Nalm6) were plated at a concentration of 2.5x105 cells/ml in 96-well flat bottom plates in triplicates. Then, 75 µg/ml D-firefly luciferin potassium salt (Biosynth) was added to the tumor cells and bioluminescence (BLI) was measured in the luminometer (Tecan infinity M200 Pro) to establish the BLI baseline. Right after, CAR/TRuC T cells were added at the indicated effector-to-target (E:T) ratio of 1:5 and incubated for 24 h at 37 °C. BLI was measured as relative light units (RLUs). RLU signals from cells treated with 1% Triton X-100 indicate maximal cell death. RLU signals from tumor cells without CAR T cells determine spontaneous cell death. Percent specific lysis (specific killing) was calculated with the following formula:
percentage specific lysis = 100 × (average spontaneous death RLU - test RLU) / (average spontaneous death RLU - average maximal death RLU). IFNγ secretion was assessed after 24 h of co-culture by ELISA using the supernatants from co-incubation of Nalm6 and CAR/TRuC T cells at E:T ration of 1:5.

**Resting protocol for CAR T cell expansion.** Two days after lentiviral transduction, 0.2x106 cells were seeded and incubated with 2, 4 or 8 µM of C10 or a corresponding 0.16% DMSO control for a total of 13 days. Fold induction of living cells was calculated between start of the treatment and end of the treatment. Subsequently, cells were washed and either left unstimulated or stimulated with Nalm6 target cells for 24 h in the absence of any compound. Afterwards, cells were stained with an anti-CD62L (Biolegend) and an anti-CD45RA (Bioscience) antibody to assess CAR T cell phenotypes by flow cytometry. In parallel, cells were stained for CD107a (Biolegend) to assess degranulation upon 3 h of target cell encounter by flow cytometry and a luciferase-based killing assay (see above) was used to determine cytotoxic activity over time. Cells were also stained with anti-CD69 (Life Technologies), anti-CD25 (Invitrogen) and anti-4-1BB (eBioscience) antibodies to assess upregulation of the activation markers by flow cytometry after 24 h of stimulation.

**Luciferase-based proliferation assay of Nalm6 cells.** 0.3x10⁶ Luciferase-expressing Nalm6 cells were seeded in the presence of 37.5 µg/ml firefly D-luciferin. Cells were either left untreated or treated with 0.33% DMSO or 8 µM of C6, C8 or C10. Bioluminescence (BLI) was measured in the luminometer (Tecan infinity M200 Pro) at 24, 48 or 72 h.

**Quantification and statistical analysis.** Statistical parameters including the exact number of samples (n) and precision measures (means and ± SD) as well as statistical significance are reported in figures and figure legends. Data sets were assessed for normal distribution with the Shapiro-Wilk normality test, which is recommended for biological samples with a sample size <50 (Ghasemi & Zahediasl, 2012). Data sets which passed the Shapiro-Wilk test were analysed with the ordinary one-way ANOVA or the two-way ANOVA with Dunnett's multiple comparison test as indicated. Data were judged to be statistically significant when P < 0.05. *P < 0.05, **P < 0.01, ***P < 0.001, ****P < 0.0001. Statistical analysis was performed in GraphPad Prism software version 10.1.1.

### References:

Allouche, A. (2012). Software News and Updates Gabedit - A Graphical User Interface for Computational Chemistry Softwares. Journal of Computational Chemistry, 32, 174-182. https://doi.org/10.1002/jcc
Baell, J. B., & Holloway, G. A. (2010). New substructure filters for removal of pan assay interference compounds (PAINS) from screening libraries and for their exclusion in bioassays. Journal of Medicinal Chemistry, 53(7), 2719-2740. https://doi.org/10.1021/jm901137j
Baeuerle, P. A., Ding, J., Patel, E., Thorausch, N., Horton, H., Gierut, J., Scarfo, I., Choudhary, R., Kiner, O., Krishnamurthy, J., Le, B., Morath, A., Baldeviano, G. C., Quinn, J., Tavares, P., Wei, Q., Weiler, S., Maus, M. V., Getts, D., ... Hofmeister, R. (2019). Synthetic TRuC receptors engaging the complete T cell receptor for potent anti-tumor response. Nature Communications, 10(1), 1-12. https://doi.org/10.1038/s41467-019-10097-0
Blanco, R., Chinestrad, P., Menna, P. L., Zinni, M. A., & Safar, F. (2020). Computación heterogénea de alta performance: Aplicación en el diseño racional de fármacos. 2020 IEEE Congreso Bienal de Argentina, ARGENCON 2020 - 2020 IEEE Biennial Congress of Argentina, ARGENCON 2020. https://doi.org/10.1109/ARGENCON49523.2020.9505328
Carrera, A. C., Paradis, H., Borlado, L. R., Roberts, T. M., & Martinez-A, C. (1995). Lck Unique Domain Influences Lck Specificity and Biological Function. The Journal of Biolgical Chemistry, 270(7), 3385-3391. https://doi.org/10.1074/jbc.270.7.3385
Daina, A., Michielin, O., & Zoete, V. (2017). SwissADME: A free web tool to evaluate pharmacokinetics, drug-likeness and medicinal chemistry friendliness of small molecules. Scientific Reports, 7(January), 1-13. https://doi.org/10.1038/srep42717
Elkamhawy, A., Ali, E. M. H., & Lee, K. (2021). New horizons in drug discovery of lymphocyte-specific protein tyrosine kinase (Lck) inhibitors: a decade review (2011-2021) focussing on structure-activity relationship (SAR) and docking insights. Journal of Enzyme Inhibition and Medicinal Chemistry, 36(1), 1574-1602. https://doi.org/10.1080/14756366.2021.1937143
Ghasemi, A., & Zahediasl, S. (2012). Normality tests for statistical analysis: A guide for non-statisticians. International Journal of Endocrinology and Metabolism, 10(2), 486-489. https://doi.org/10.5812/ijem.3505
Gkourtsa, A., van den Burg, J., Avula, T., Hochstenbach, F., & Distel, B. (2016). Binding of a proline-independent hydrophobic motif by the Candida albicans Rvs167-3 SH3 domain. Microbiological Research, 190, 27-36. https://doi.org/10.1016/j.micres.2016.04.018
Hartl, F. A., Beck-Garcia, E., Woessner, N. M., Flachsmann, L. J., Cárdenas, R. M.-H. V., Brandl, S. M., Taromi, S., Fiala, G. J., Morath, A., Mishra, P., Yousefi, O. S., Zimmermann, J., Hoefflin, N., Köhn, M., Wöhrl, B. M., Zeiser, R., Schweimer, K., Günther, S., Schamel, W. W., & Minguet, S. (2020). Noncanonical binding of Lck to CD3ε promotes TCR signaling and CAR function. Nature Immunology, 21(8), 902-913. https://doi.org/10.1038/s41590-020-0732-3
Horkova, V., Drobek, A., Paprckova, D., Niederlova, V., Prasai, A., Uleri, V., Glatzova, D., Kraller, M., Cesnekova, M., Janusova, S., Salyova, E., Tsyklauri, O., Kadlecek, T. A., Krizova, K., Platzer, R., Schober, K., Busch, D. H., Weiss, A., Huppa, J. B., & Stepanek, O. (2023). Unique roles of co-receptor-bound LCK in helper and cytotoxic T cells. Nature Immunology, 24(January). https://doi.org/10.1038/s41590-022-01366-0
Kang, H., Freund, C., Duke-Cohan, J. S., Musacchio, A., Wagner, G., & Rudd, C. E. (2000). SH3 domain recognition of a proline-independent tyrosine-based RKxxYxxY motif in immune cell adaptor SKAP55. EMBO Journal, 19(12), 2889-2899. https://doi.org/10.1093/emboj/19.12.2889
Li, Guo, X., Shi, X., Li, C., Wu, W., Yan, C., Wang, H., Li, H., & Xu, C. (2017). Ionic CD3-Lck interaction regulates the initiation of T-cell receptor signaling. Proceedings of the National Academy of Sciences of the United States of America, 114(29), E5891-E5899. https://doi.org/10.1073/pnas.1701990114
Lipinski, C. A., Lombardo, F., Dominy, B. W., & Feeney, P. J. (2012). Experimental and computational approaches to estimate solubility and permeability in drug discovery and development settings. Advanced Drug Delivery Reviews, 64(SUPPL.), 4-17. https://doi.org/10.1016/j.addr.2012.09.019
López-Cantillo, G., Urueña, C., Camacho, B. A., & Ramírez-Segura, C. (2022). CAR-T Cell Performance: How to Improve Their Persistence? In Frontiers in Immunology (Vol. 13). Frontiers Media S.A. https://doi.org/10.3389/fimmu.2022.878209
Martin, M. W., & Machacek, M. R. (2010). Update on lymphocyte specific kinase inhibitors: a patent survey. Expert Opinion on Therapeutic Patents, 1573-1593. https://doi.org/10.1517/13543776.2010.517749
Minguet S., Maus M., and Schamel W. (2025). From TCR fundamental research to innovative chimeric antigen receptor design. Nature reviews immunology, 25(3), 212-224. doi: 10.1038/s41577-024-01093. Epub 2024 Oct 21.
Morris, M. G., Huey, R., Lindstrom, W., Sanner, F. M., Belew, K. R., Goodsell, S. D., & Olson, J. A. (2009). AutoDock4 and AutoDockTools4: Automated Docking with Selective Receptor Flexibility. Journal of Computational Chemistry, 30, 2785-2791. https://doi.org/10.1002/jcc.21256 https://doi.org/10.1126/scitranslmed.abp9675
Salentin, S., Schreiber, S., Haupt, V. J., Adasme, M. F., & Schroeder, M. (2015). PLIP: Fully automated protein-ligand interaction profiler. Nucleic Acids Research, 43(W1), W443-W447. https://doi.org/10.1093/nar/gkv315
Salter, A. I., Rajan, A., Kennedy, J. J., Ivey, R. G., Shelby, S. A., Leung, I., Templeton, M. L., Muhunthan, V., Voillet, V., Sommermeyer, D., Whiteaker, J. R., Gottardo, R., Veatch, S. L., Paulovich, A. G., & Riddell, S. R. (2021). Comparative analysis of TCR and CAR signaling informs CAR designs with superior antigen sensitivity and in vivo function. Science Signaling, 14(697), eabe2606. https://doi.org/10.1126/scisignal.abe2606
Sicheri, F., & Kuriyant, J. (1997). Structures of Src-family tyrosine kinases. Current Opinion in Structural Biology, 7, 777-785.
Stachlewitz, R. F., Hart, M. A., Bettencourt, B., Kebede, T., Schwartz, A., Ratnofsky, S. E., Calderwood, D. J., Waegell, W. O., & Hirst, G. C. (2005). A-770041, a novel and selective small-molecule inhibitor of Lck, prevents heart allograft rejection. Journal of Pharmacology and Experimental Therapeutics, 315(1), 36-41. https://doi.org/10.1124/jpet.105.089169
Velasco Cárdenas, R. M. H., Brandl, S., Meléndez, A. V., Schlaak, E. A., Buschky, A., Peters, T., Beier, F., Serrels, B., Taromi, S., Raute, K., Hauri, S., Gstaiger, M., Lassman, S., Huppa, J. B., Boerries, M., Andrieux, G., Bengsch, B., Schamel, W. W., & Minguet, S. (2023). Harnessing CD3 diversity to optimise CAR T cells. Nature Immunol. https://doi.org/10.1038/s41590-023-01658-z
Woessner, N. M., Brandl, S. M., Hartmann, S., Schamel, W. W., Hartl, F. A., & Minguet, S. (2024). Phospho-mimetic CD3 e variants prevent TCR and CAR signaling. Front Immunol, 15(May), 1-14. https://doi.org/10.3389/fimmu.2024.1392933
Wu, W., Zhou, Q., Masubuchi, T., Shi, X., Li, H., Xu, X., Huang, M., Meng, L., He, X., Zhu, H., Gao, S., Zhang, N., Jing, R., Sun, J., Wang, H., Hui, E., Wong, C. C., & Xu, C. (2020). Multiple Signaling Roles of CD3ε and Its Application in CAR-T Cell Therapy. Cell, 182(4), 855-871.e23. https://doi.org/10.1016/j.cell.2020.07.018
Xu, W., Harrison, S. C., & Eck, M. J. (1997). Three-dimensional structure of the tyrosine kinase c-Src. Nature, 385(6617), 595-602. https://doi.org/10.1038/385595a0
Zhang, Y., & Sanner, M. F. (2019). AutoDock CrankPep: Combining folding and docking to predict protein-peptide complexes. Bioinformatics, 35(24), 5121-5127. https://doi.org/10.1093/bioinformatics/btz459
Zhu, X., Kim, J. L., Newcomb, J. R., Rose, P. E., Stover, D. R., Toledo, L. M., Zhao, H., & Morgenstern, K. A. (1999). Structural analysis of the lymphocyte-specific kinase Lck in complex with non-selective and Src family selective kinase inhibitors. Structure, 7(6), 651-661. https://doi.org/10.1016/S0969-2126(99)80086-0

## Claims

1. A small molecule binding to the SH3 domain of the lymphocyte-specific protein tyrosine kinase (LCK), wherein the small molecule reduces or prevents the interaction of LCK with CD3ε, for use in therapy.

2. A small molecule binding to the SH3 domain of the lymphocyte-specific protein tyrosine kinase (LCK), wherein the small molecule reduces or prevents the interaction of LCK with CD3ε, for use in treating a T cell involving autoimmune or inflammatory disease.

3. The small molecule for use according to claim 2, wherein the T cell involving autoimmune or inflammatory disease is selected from the group consisting of rheumatoid arthritis, psoriasis, multiple sclerosis, atherosclerosis, graft-versus-host disease, allograft rejection, asthma, type 1 diabetes mellitus, vitiligo, celiac disease, Graves' disease, Hashimoto's thyroiditis, Addison's disease, systemic lupus erythematosus, Sjögren's Syndrome, systemic sclerosis, inflammatory bowel disease including Crohn's Disease and ulcerative colitis, Lichen Planus, atopic dermatitis, autoimmune hemolytic anemia, immune thrombocytopenic purpura (ITP), alopecia areata, autoimmune uveitis, sarcoidosis and ankylosing spondylitis.

4. A small molecule binding to the SH3 domain of the lymphocyte-specific protein tyrosine kinase (LCK), wherein the small molecule reduces or prevents the interaction of LCK with CD3ε, for use in treating a side effect associated with a T cell-based immunotherapy, preferably wherein the T cell-based immunotherapy is a chimeric antigen receptor (CAR) T cell immunotherapy or a TCR-based chimeric receptor T cell immunotherapy.

5. The small molecule for use according to claim 4, wherein the side effect associated with a T cell-based immunotherapy is selected from cytokine release syndrome, neurotoxicity and graft-versus-host disease.

6. The small molecule for use according to any one of claims 1 to 5, wherein the small molecule exhibits (i) an IC50 of < about 20 µM in an *in vitro* T cell proliferation inhibition assay and/or (ii) a reduction of at least about 30% of the LCK-TCR interaction in a proximity ligation assay (PLA) or in a pull down (PD) assay.

7. The small molecule for use according to any one of claims 1 to 6, wherein the small molecule exhibits an IC50 of < about 10 µM in an *in vitro* T cell proliferation inhibition assay and a reduction of at least about 60% of the LCK-TCR interaction in a proximity ligation assay (PLA) or in a pull down (PD) assay.

8. The small molecule for use according to any one of claims 1 to 6, wherein the small molecule is selected from the group consisting of a compound of formula (I) 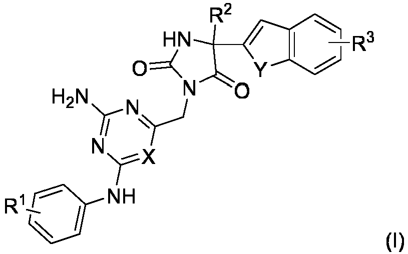
or a stereoisomer, tautomer, N-oxide, pharmaceutically acceptable salt, or hydrate thereof, wherein
X is N, or CH;
Y is O, S, or N-R^{N};
wherein R^{N} is H or C₁-C₆-alkyl; and
R¹, R² and R³ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 3- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R⁴;
wherein R⁴ is halogen, C₁-C₄-alkyl, or C₁-C₄-haloalkyl;
a compound of formula (II) 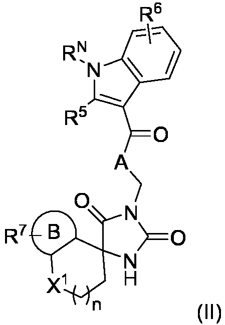
or a stereoisomer, tautomer, *N*-oxide, pharmaceutically acceptable salt, or hydrate thereof, wherein
n is 0 to 2;
A is absent or 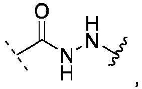 wherein the dashed line represents the connection to the methylene group and the curled line represents the connection to the carbonyl group;
ring B is a five- or six-membered monocyclic heteroaryl or a five- or six-membered monocyclic aryl;
X¹ is CH₂ or O; and
R⁵, R⁶ and R⁷ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 5- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R⁸;
wherein R⁸ is halogen, C₁-C₄-alkyl, or C₁-C₄-haloalkyl; and
R^{N} is H or C₁-C₄-alkyl; and
a compound of formula (III) 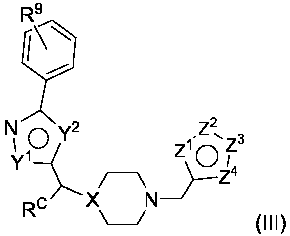
or a stereoisomer, tautomer, *N*-oxide, pharmaceutically acceptable salt, or hydrate thereof, wherein
X is N or CR^{X};
wherein R^{X} is H or C₁₋₃-alkyl;
and
R^{C} is H or C₁₋₃-alkyl;
or R^{X} and R^{C} together form cyclopropyl; and
Y¹ and Y² are independently selected from N or O,
provided that not both Y¹ and Y² are N; and
provided not both Y¹ and Y² are O; and
Z¹, Z², Z³ and Z⁴ are independently selected from N, CR^{R} or O;
provided that not two Z¹-Z⁴ are N; and
provided that not more than one of Z¹-Z⁴ is O and at least one of Z¹-Z⁴ is CR^{R};
wherein R^{R} is C₁₋₆-alkyl, furanyl, phenyl, or benzyl,
wherein both phenyl or benzyl are optionally substituted with R¹⁰; and
R⁹ and R¹⁰ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 3- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or
different substituents R¹¹;
wherein R¹¹ is halogen, C₁-C₄-alkyl, or C₁-C₄-haloalkyl.

9. The small molecule for use according to any one of claims 1 to 6 and 8, wherein the small molecule is selected from the group consisting of 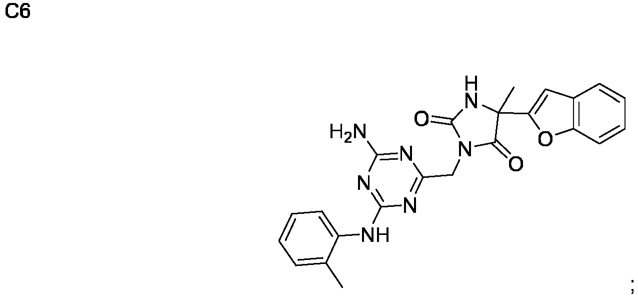 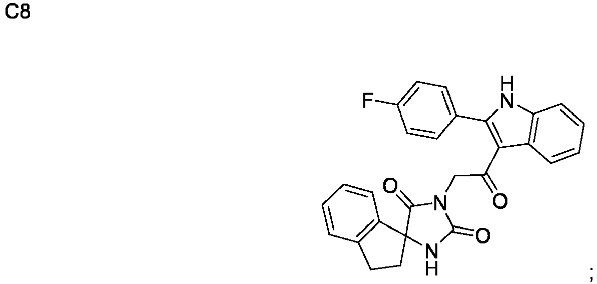 and

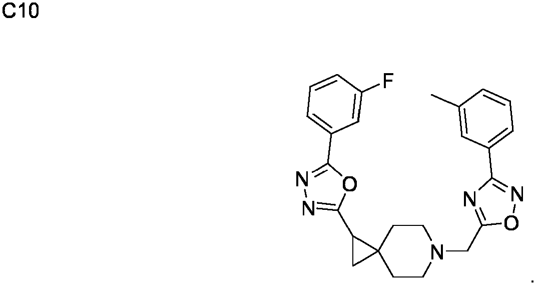

10. An *in vitro* method of manufacturing a T cell comprising a chimeric antigen receptor (CAR) or a TCR-based chimeric receptor, comprising the step of contacting the T cell with a small molecule binding to the SH3 domain of the lymphocyte-specific protein tyrosine kinase (LCK), wherein the small molecule reduces or prevents the interaction of LCK with CD3ε.

11. Use of a small molecule binding to the SH3 domain of the lymphocyte-specific protein tyrosine kinase (LCK), wherein the small molecule reduces or prevents the interaction of LCK with CD3ε, to prevent or reduce early exhaustion of a T cell, optionally a T cell comprising a chimeric antigen receptor (CAR) or a TCR-based chimeric receptor.

12. The method according to claim 10 or the use according to claim 11, wherein the small molecule exhibits an IC50 of < about 20 µM in an *in vitro* T cell proliferation inhibition assay and/or a reduction of at least about 30% of the LCK-TCR interaction in a PLA or in a pull down (PD) assay.

13. The method according to claim 10 and 12 or the use according to claim 11 and 12, wherein the small molecule exhibits an IC50 of < about 10 µM in an *in vitro* T cell proliferation inhibition assay and a reduction of at least about 60% of the LCK-TCR interaction in a PLA or in a pull down (PD) assay.

14. The method according to claim 10, 12 and 13 or the use according to claim 11 to 13, wherein the small molecule is selected from the group consisting of a compound of formula (I) 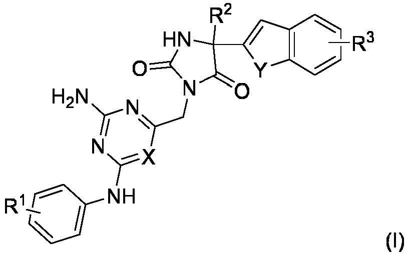
or a stereoisomer, tautomer, *N*-oxide, pharmaceutically acceptable salt, or hydrate thereof, wherein
X is N, or CH;
Y is O, S, or N-R^{N};
wherein R^{N} is H or C₁-C₆-alkyl; and
R¹, R² and R³ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 3- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R⁴;
wherein R⁴ is halogen, C₁-C₄-alkyl, or C₁-C₄-haloalkyl;
a compound of formula (II) 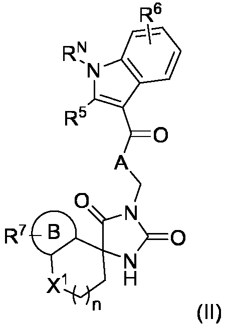
or a stereoisomer, tautomer, *N*-oxide, pharmaceutically acceptable salt, or hydrate thereof, wherein
n is 0 to 2;
A is absent or 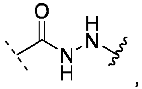 wherein the dashed line represents the connection to the methylene group and the curled line represents the connection to the carbonyl group;
ring B is a five- or six-membered monocyclic heteroaryl or a five- or six-membered monocyclic aryl;
X¹ is CH₂ or O; and
R⁵, R⁶ and R⁷ are independently H, halogen, CN, C₁-C₄-alkyl, C₁-C₄-alkoxy, NO₂, C₁-C₄-perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 5- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R⁸;
wherein R⁸ is halogen, C₁-C₄-alkyl, or C₁-C₄-haloalkyl; and
R^{N} is H or C₁-C₄-alkyl; and
a compound of formula (III) 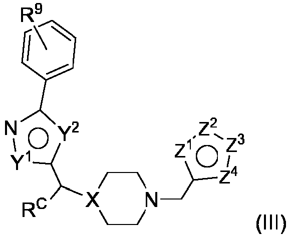
or a stereoisomer, tautomer, *N*-oxide, pharmaceutically acceptable salt, or hydrate thereof, wherein
X is N or CR^{X};
wherein R^{X} is H or C₁₋₃-alkyl;
and
R^{C} is H or C₁₋₃-alkyl;
or R^{X} and R^{C} together form cyclopropyl; and
Y¹ and Y² are independently selected from N or O,
provided that not both Y¹ and Y² are N; and
provided not both Y¹ and Y² are O; and
Z¹, Z², Z³ and Z⁴ are independently selected from N, CR^{R} or O;
provided that not two Z¹-Z⁴ are N; and
provided that not more than one of Z¹-Z⁴ is O and at least one of Z¹-Z⁴ is CR^{R};
wherein R^{R} is C₁₋₆-alkyl, furanyl, phenyl, or benzyl,
wherein both phenyl or benzyl are optionally substituted with R¹⁰; and
R⁹ and R¹⁰ are independently H, halogen, CN, C₁-C₄-alkyl, C₁-C₄-alkoxy, NO₂, C₁-C₄- perfluoroalkyl, C(=O)O-C₁-C₄-alkyl, C₁-C₄-aminoalkyl, S(=O)-C₁-C₄-alkyl, S(=O)₂-C₁-C₄-alkyl, C(=O)-C₁-C₄-alkyl, C₁-C₆-thioalkyl,
or a 3- to 6-membered saturated, partially or fully unsaturated or aromatic carbocyclyl or carbocyclyloxy and wherein each substitutable atom in the aforementioned carbocyclyl or carbocyclyloxy rings is independently unsubstituted or substituted with one or more, same or different substituents R¹¹;
wherein R¹¹ is halogen, C₁-C₄-alkyl, or C₁-C₄-haloalkyl.

15. The method according to claim 10, 12, 13 and 14 or the use according to claim 11 to 14, wherein the small molecule is selected from the group consisting of
